(19) **Europäisches Patentamt / European Patent Office / Office européen des brevets**

(11) **EP 4 127 243 B1**

(12) **EUROPEAN PATENT SPECIFICATION**

(45) Date of publication and mention of the grant of the patent:
**22.11.2023 Bulletin 2023/47**

(21) Application number: **21711274.7**

(22) Date of filing: **16.03.2021**

(51) International Patent Classification (IPC):
**C12Q 1/6886** *(2018.01)*

(52) Cooperative Patent Classification (CPC):
**C12Q 1/6886; C12Q 2600/106; C12Q 2600/158**

(86) International application number:
**PCT/EP2021/056582**

(87) International publication number:
**WO 2021/190983 (30.09.2021 Gazette 2021/39)**

(54) **PREDICTION OF RADIOTHERAPY RESPONSE FOR PROSTATE CANCER SUBJECT BASED ON IMMUNE CHECKPOINT GENES**

VORHERSAGE DER RADIOTHERAPIEREAKTION FÜR PROSTATAKREBSPATIENT AUF BASIS VON IMMUN-CHECKPOINT-GENEN

PRÉDICTION DE LA RÉPONSE À LA RADIOTHÉRAPIE POUR UN SUJET ATTEINT D'UN CANCER DE LA PROSTATE BASÉE SUR DES GÈNES DE POINT DE CONTRÔLE IMMUNITAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2020 EP 20164809**
**24.03.2020 EP 20165097**

(43) Date of publication of application:
**08.02.2023 Bulletin 2023/06**

(73) Proprietor: **Koninklijke Philips N.V.**
**5656 AG Eindhoven (NL)**

(72) Inventors:
• **STOFFELS, Monique**
**5656 AE Eindhoven (NL)**
• **HOFFMANN, Ralf, Dieter**
**5656 AE Eindhoven (NL)**
• **VAN LIESHOUT, Ron, Martinus, Laurentius**
**5656 AE Eindhoven (NL)**

(74) Representative: **Philips Intellectual Property & Standards**
**High Tech Campus 52**
**5656 AG Eindhoven (NL)**

(56) References cited:
**WO-A1-2017/198617    WO-A2-2019/028285**

**CA-A1- 3 072 061    US-A1- 2019 249 258**

• **Anonymous: "Affymetrix GeneChip Human Genome U133 Array Set HGU133A", NCBI, GEO, Platform GPL96, 11 March 2002 (2002-03-11), pages 1-511, XP055546924, Retrieved from the Internet: URL:https://www.ncbi.nlm.nih.gov/geo/query /acc.cgi?acc=GPL96 [retrieved on 2019-01-24]**
• **ROBERT B. DEN ET AL: "Genomic Classifier Identifies Men With Adverse Pathology After Radical Prostatectomy Who Benefit From Adjuvant Radiation Therapy", JOURNAL OF CLINICAL ONCOLOGY, vol. 33, no. 8, 10 March 2015 (2015-03-10) , pages 944-951, XP055592410, US ISSN: 0732-183X, DOI: 10.1200/JCO.2014.59.0026 cited in the application**
• **GRASSBERGER CLEMENS ET AL: "Assessing the interactions between radiotherapy and antitumour immunity", NATURE REVIEWS CLINICAL ONCOLOGY, NATURE, NY, US, vol. 16, no. 12, 26 June 2019 (2019-06-26) , pages 729-745, XP036928179, ISSN: 1759-4774, DOI: 10.1038/S41571-019-0238-9 [retrieved on 2019-06-26] cited in the application**

**(Cont. next page)**

Remarks:

The complete document including Reference Table(s) and the Sequence Listing(s) can be downloaded from the EPO website

**Description**

FIELD OF THE INVENTION

The invention relates to a method of predicting a response of a prostate cancer subject to radiotherapy. Moreover, the invention relates to a diagnostic kit, to a use of the kit in a method of predicting a response of a prostate cancer subject to radiotherapy, to a use of a gene expression profile for each of two or more immune checkpoint genes in radiotherapy prediction for a prostate cancer subject, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize. Prostate Cancer (PCa) is the second most commonly-occurring non-skin malignancy in men, with an estimated 1.3 million new cases diagnosed and 360,000 deaths world-wide in 2018 (see Bray F. et al., "Global cancer statistics 2018: GLOBOCAN estimates of incidence and mortality worldwide for 36 cancers in 185 countries", CA Cancer J Clin, Vol. 68, No. 6, pages 394-424, 2018). In the US, about 90% of the new cases concern localized cancer, meaning that metastases have not yet been formed (see ACS (American Cancer Society), "Cancer Facts & Figures 2010", 2010).

For the treatment of primary localized prostate cancer, several radical therapies are available, of which surgery (radical prostatectomy, RP) and radiation therapy (RT) are most commonly used. RT is administered via an external beam or via the implantation of radioactive seeds into the prostate (brachytherapy) or a combination of both. It is especially preferable for patients who are not eligible for surgery or have been diagnosed with a tumour in an advanced localized or regional stage. Radical RT is provided to up to 50% of patients diagnosed with localized prostate cancer in the US (see ACS, 2010, ibid).

After treatment, prostate cancer antigen (PSA) levels in the blood are measured for disease monitoring. An increase of the blood PSA level provides a biochemical surrogate measure for cancer recurrence or progression. However, the variation in reported biochemical progression-free survival (bPFS) is large (see Grimm P. et al., "Comparative analysis of prostate-specific antigen free survival outcomes for patients with low, intermediate and high risk prostate cancer treatment by radical therapy. Results from the Prostate Cancer Results Study Group", BJU Int, Suppl. 1, pages 22-29, 2012). For many patients, the bPFS at 5 or even 10 years after radical RT may lie above 90%. Unfortunately, for the group of patients at medium and especially higher risk of recurrence, the bPFS can drop to around 40% at 5 years, depending on the type of RT used (see Grimm P. et al., 2012, ibid).

A large number of the patients with primary localized prostate cancer that are not treated with RT will undergo RP (see ACS, 2010, ibid). After RP, an average of 60% of patients in the highest risk group experience biochemical recurrence after 5 and 10 years (see Grimm P. et al., 2012, ibid). In case of biochemical progression after RP, one of the main challenges is the uncertainty whether this is due to recurring localized disease, one or more metastases or even an indolent disease that will not lead to clinical disease progression (see Dal Pra A. et al., "Contemporary role of postoperative radiotherapy for prostate cancer", Transl Androl Urol, Vo. 7, No. 3, pages 399-413, 2018, and Herrera F.G. and Berthold D.R., "Radiation therapy after radical prostatectomy: Implications for clinicians", Front Oncol, Vol. 6, No. 117, 2016). RT to eradicate remaining cancer cells in the prostate bed is one of the main treatment options to salvage survival after a PSA increase following RP. The effectiveness of salvage radiotherapy (SRT) results in 5-year bPFS for 18% to 90% of patients, depending on multiple factors (see Herrera F.G. and Berthold D.R., 2016, ibid, and Pisansky T.M. et al., "Salvage radiation therapy dose response for biochemical failure of prostate cancer after prostatectomy - A multi-institutional observational study", Int J Radiat Oncol Biol Phys, Vol. 96, No. 5, pages 1046-1053, 2016).

It is clear that for certain patient groups, radical or salvage RT is not effective. Their situation is even worsened by the serious side effects that RT can cause, such as bowel inflammation and dysfunction, urinary incontinence and erectile dysfunction (see Resnick M.J. et al., "Long-term functional outcomes after treatment for localized prostate cancer", N Engl J Med, Vol. 368, No. 5, pages 436-445, 2013, and Hegarty S.E. et al., "Radiation therapy after radical prostatectomy for prostate cancer: Evaluation of complications and influence of radiation timing on outcomes in a large, population-based cohort", PLoS One, Vol. 10, No. 2, 2015). In addition, the median cost of one course of RT based on Medicare reimbursement is $ 18,000, with a wide variation up to about $ 40,000 (see Paravati A.J. et al., "Variation in the cost of radiation therapy among medicare patients with cancer", J Oncol Pract, Vol. 11, No. 5, pages 403-409, 2015). These figures do not include the considerable longitudinal costs of follow-up care after radical and salvage RT.

An improved prediction of effectiveness of RT for each patient, be it in the radical or the salvage setting, would improve therapy selection and potentially survival. This can be achieved by 1) optimizing RT for those patients where RT is predicted to be effective (e.g., by dose escalation or a different starting time) and 2) guiding patients where RT is predicted not to be effective to an alternative, potentially more effective form of treatment. Further, this would reduce suffering for those patients who would be spared ineffective therapy and would reduce costs spent on ineffective therapies.

Numerous investigations have been conducted into measures for response prediction of radical RT (see Hall W.A. et al., "Biomarkers of outcome in patients with localized prostate cancer treated with radiotherapy", Semin Radiat Oncol, Vol. 27, pages 11-20, 2016, and Raymond E. et al., "An appraisal of analytical tools used in predicting clinical outcomes following radiation therapy treatment of men with prostate cancer: A systematic review", Radiat Oncol, Vol. 12, No. 1, page 56, 2017) and SRT (see Herrera F.G. and Berthold D.R., 2016, ibid). Many of these measures depend on the concentration of the blood-based biomarker PSA. Metrics investigated for prediction of response before start of RT (radical as well as salvage) include the absolute value of the PSA concentration, its absolute value relative to the prostate volume, the absolute increase over a certain time and the doubling time. Other frequently considered factors are the Gleason score and the clinical tumour stage. For the SRT setting, additional factors are relevant, e.g., surgical margin status, time to recurrence after RP, pre-/peri-surgical PSA values and clinico-pathological parameters.

Although these clinical variables provide limited improvements in patient stratification in various risk groups, there is a need for better predictive tools.

A wide range of biomarker candidates in tissue and bodily fluids has been investigated, but validation is often limited and generally demonstrates prognostic information and not a predictive (therapy-specific) value (see Hall W.A. et al., 2016, ibid). A small number of gene expression panels is currently being validated by commercial organizations. One or a few of these may show predictive value for RT in future (see Dal Pra A. et al., 2018, ibid).

In conclusion, a strong need for better prediction of response to RT remains, for primary prostate cancer as well as for the post-surgery setting.

The Affymetrix GeneChip Human Genome U133 Array Set HG-U133A includes over 1,000,000 unique oligonucleotide features covering more than 39,000 transcript variants, which in turn represent greater than 33,000 human genes.

Den R.B. et al., "Genomic Classifier Identifies Men With Adverse Pathology After Radical Prostatectomy Who Benefit From Adjuvant Radiation Therapy", Journal of Clinical Oncology, Vol. 33, No. 8, pages 944-951, 2015, starts out from the observation that the optimal timing of postoperative radiotherapy (RT) after radical prostatectomy (RP) is unclear. It finds that a genomic classifier (GC) is prognostic for the development of clinical metastasis beyond routine clinical and pathologic features.

Grassberger C. et al., "Assessing the interactions between radiotherapy and antitumour immunity", Nature Reviews Clinical Oncology, Vol. 16, No. 12, pages 729-745, 2019, discusses modelling approaches that have been developed to predict the interaction of immunotherapy with radiotherapy, and highlights how they could be combined with biomarkers of antitumour immunity to optimize radiotherapy regimens and maximize their synergy with immunotherapy.

WO 2019/028285 A2 discloses methods, systems, and kits for the diagnosis, prognosis and the determination of cancer progression of prostate cancer in a subject. In particular, the disclosure relates to the use of immune cell-specific gene expression in determining prognosis and identifying individuals in need of treatment for prostate cancer who will be responsive to radiation therapy.

SUMMARY OF THE INVENTION

It is an objective of the invention to provide a method of predicting a response of a prostate cancer subject to radiotherapy, which allows to make better treatment decisions. It is a further objective of the invention to provide a diagnostic kit, a use of the kit in a method of predicting a response of a prostate cancer subject to radiotherapy, a use of a gene expression profile for each of two or more immune checkpoint genes in radiotherapy prediction for a prostate cancer subject, and a corresponding computer program product.

In a first aspect of the present invention, a method of predicting a response of a prostate cancer subject to radiotherapy is presented, comprising:

- determining or receiving the result of a determination of a gene expression profile for each of two or more, for example, 2, 3, 4 or all, immune checkpoint genes selected from the group consisting of: LAG3, LGALS9, TDO2, TMIGD2, and VTCN1, said gene expression profiles being determined in a biological sample obtained from the subject,
- determining, preferably by a processor, the prediction of the radiotherapy response based on the gene expression profiles for the two or more immune checkpoint genes, and
- optionally, providing the prediction or a therapy recommendation based on the prediction to a medical caregiver or the subject.

In recent years, the importance of the immune system in cancer inhibition as well as in cancer initiation, promotion and metastasis has become very evident (see Mantovani A. et al., "Cancer-related inflammation", Nature, Vol. 454, No.

7203, pages 436-444, 2008, and Giraldo N.A. et al., "The clinical role of the TME in solid cancer", Br J Cancer, Vol. 120, No. 1, pages 45-53, 2019). The immune cells and the molecules they secrete form a crucial part of the tumour microenvironment and most immune cells can infiltrate the tumour tissue. The immune system and the tumour affect and shape one another. Thus, anti-tumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (see Giraldo N.A. et al., 2019, ibid).

The principles described above with regard to the role of the immune system in cancer in general also apply to prostate cancer. Chronic inflammation has been linked to the formation of benign as well as malignant prostate tissue (see Hall W.A. et al., 2016, ibid) and most prostate cancer tissue samples show immune cell infiltrates. The presence of specific immune cells with a pro-tumour effect has been correlated with worse prognosis, while tumours in which natural killer cells were more activated showed better response to therapy and longer recurrence-free periods (see Shiao S.L. et al., "Regulation of prostate cancer progression by tumor microenvironment", Cancer Lett, Vol. 380, No. 1, pages 340-348, 2016).

While a therapy will be influenced by the immune components of the tumour microenvironment, RT itself extensively affects the make-up of these components (see Barker H.E. et al., "The tumor microenvironment after radiotherapy: Mechanisms of resistance or recurrence", Nat Rev Cancer, Vol. 15, No. 7, pages 409-425, 2015). Because suppressive cell types are comparably radiation-insensitive, their relative numbers will increase. Counteractively, the inflicted radiation damage activates cell survival pathways and stimulates the immune system, triggering inflammatory responses and immune cell recruitment. Whether the net effect will be tumour-promoting or tumour-suppressing is as yet uncertain, but its potential for enhancement of cancer immunotherapies is being investigated.

The present invention is based on the idea that, since the status of the immune system and of the immune microenvironment have an impact on therapy effectiveness, the ability to identify markers predictive for this effect might help to be better able to predict overall RT response.

Immune checkpoint molecules play a central role in the regulation of the immune response. Based on the interaction between and the combination of a receptor and a ligand, downstream signaling can be either stimulatory or inhibitory. Multiple checkpoint molecules are involved in the activation or suppression of T-cells in the TME, which in their turn play a role in for example clearing of tumour cells.

Upon recognition, the immune system builds an immune response to clear tumour cells. Clonal expansion, activation, and localization of T-cells that recognize tumour-specific antigens presented on the surface of tumour cells are crucial for tumour cell elimination. Once completed, this response must be dampened to prevent collateral damage to surrounding tissue. Immune checkpoints are crucial for self-tolerance, a way in which the immune system is prevented from attacking cells indiscriminately.

Depending on the type immune checkpoint interaction, downstream signaling may lead to promotion or inhibition of T-cell activation. A single T-cell expresses multiple receptors (and ligands) that can form an immune checkpoint with a ligand on another cell (TABLES 1a and 1b), and the balance of overall positive and negative downstream signals will dictate whether a T-cell will be inhibited in its tumour killing function or not.

Tumour cells often express inhibitory checkpoint molecules to evade elimination by the immune system. Therefore, inhibitory checkpoint molecules are targeted in cancer immunotherapies for several types of cancer. RT can modulate the anticancer immune response, including the abscopal effect, and it is recognized that presence and function of the infiltrate can be used to predict therapy outcome. Immune checkpoint inhibition therapy is already applied in several cancer types, and also for prostate cancer clinical relevance is under investigation. For example, although known as a bad marker for immune therapy response, protein expression of PDL1 has been correlated with high risk prostate cancer, and is also an independent biomarker in the prognosis of high-risk prostate cancer received ART after RP (see Li H. et al., "The immune checkpoint regulator PDL1 is an independent prognostic biomarker for biochemical recurrence in prostate cancer patients following adjuvant hormonal therapy", J Cancer, Vol. 10, No. 14, pages 3102-3111, 2019). Nevertheless, as described in a recent meta-analysis, the field of prostate cancer, despite being a leader in employing clinical tumour immunology tools, has fallen behind in providing solid proofs of success for clinical impact of immune checkpoint inhibitors in metastatic castrate resistant prostate cancer (see Taghizadeh H. et al., "Immune checkpoint inhibitors in mCRPC - Rationales, challenges and perspectives", Oncoimmunology, Vol. 8, No. 11, 2019). Therefore, although suggestive, it is extremely difficult to conclude based on literature which members of the immune checkpoint molecules might be specifically predictive for the response of prostate cancer patients to radical RT or SRT.

The identified immune checkpoint genes LAG3, LGALS9, TDO2, TMIGD2, and VTCN1 were identified as follows: A group of 538 prostate cancer patients were treated with RP and the prostate cancer tissue was stored. A number of these patients experienced biochemical recurrence and was treated with SRT. For 151 of these patients, the RNA expressed in the originally stored prostate cancer tissue was analysed using RNA sequencing. The mRNA expression of immune checkpoint related genes was compared for the 26 out of 151 patients that died due to prostate cancer, versus the 125 out of 151 patients who survived. For the five molecules LAG3, LGALS9, TDO2, TMIGD2, and VTCN1, the expression level was significantly different for the survivors, suggesting that they have value in the prediction of survival after SRT. Several of these five molecules were found to correlate with prostate cancer, be it in different settings, as described further below.

The term "LAG3" refers to the Lymphocyte-Activation gene 3 (Ensembl: ENSG00000089692), for example, to the sequence as defined in NCBI Reference Sequence NM_002286, specifically, to the nucleotide sequence as set forth in SEQ ID NO:1, which corresponds to the sequence of the above indicated NCBI Reference Sequence of the LAG3 transcript, and also relates to the corresponding amino acid sequence for example as set forth in SEQ ID NO:2, which corresponds to the protein sequence defined in NCBI Protein Accession Reference Sequence NP_002277 encoding the LAG3 polypeptide.

The term "LAG3" also comprises nucleotide sequences showing a high degree of homology to LAG3, e.g., nucleic acid sequences being at least 75%, 80%, 85%, 90%, 91%, 92%, 93%, 94%, 95%, 96%, 97%, 98% or 99% identical to the sequence as set forth in SEQ ID NO:1 or amino acid sequences being at least 75%, 80%, 85%, 90%, 91%, 92%, 93%, 94%, 95%, 96%, 97%, 98% or 99% identical to the sequence as set forth in SEQ ID NO:2 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:2 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 1.

The term "LGALS9" refers to the Galectin-9 gene (Ensembl: ENSG00000168961), for example, to the sequence as defined in NCBI Reference Sequence NM_002308 or in NCBI Reference Sequence NM_009587 or in NCBI Reference Sequence NM_001330163, specifically, to the nucleotide sequences as set forth in SEQ ID NO:3 or in SEQ ID NON or in SEQ ID NO:5, which correspond to the sequences of the above indicated NCBI Reference Sequences of the LGALS9 transcript, and also relates to the corresponding amino acid sequences for example as set forth in SEQ ID NO:6 or in SEQ ID NO:7 or in SEQ ID NO:8, which correspond to the protein sequences defined in NCBI Protein Accession Reference Sequence NP_002299 and in NCBI Protein Accession Reference Sequence NP_033665 and in NCBI Protein Accession Reference Sequence NP_001317092 encoding the LGALS9 polypeptide.

The term "LGALS9" also comprises nucleotide sequences showing a high degree of homology to LGALS9, e.g., nucleic acid sequences being at least 75%, 80%, 85%, 90%, 91%, 92%, 93%, 94%, 95%, 96%, 97%, 98% or 99% identical to the sequence as set forth in SEQ ID NON or in SEQ ID NON or in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or in SEQ ID NO:7 or in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or in SEQ ID NON or in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NON or in SEQ ID NO:4 or in SEQ ID NO:5.

The term "TDO2" refers to the Tryptophan 2,3-Dioxygenase gene (Ensembl: ENSG00000151790), for example, to the sequence as defined in NCBI Reference Sequence NM_005651, specifically, to the nucleotide sequence as set forth in SEQ ID NO:9, which corresponds to the sequence of the above indicated NCBI Reference Sequence of the TDO2 transcript, and also relates to the corresponding amino acid sequence for example as set forth in SEQ ID NO:10, which corresponds to the protein sequence defined in NCBI Protein Accession Reference Sequence NP_005642 encoding the TDO2 polypeptide.

The term "TDO2" also comprises nucleotide sequences showing a high degree of homology to TDO2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO:10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "TMIGD2" refers to the Transmembrane and Immunoglobulin Domain Containing 2 gene (Ensembl: ENSG00000167664), for example, to the sequence as defined in NCBI Reference Sequence NM_144615 or in NCBI Reference Sequence NM_001169126 or in NCBI Reference Sequence NM_001308232, specifically, to the nucleotide sequences as set forth in SEQ ID NO:11 or in SEQ ID NO:12 or in SEQ ID NO:13, which correspond to the sequences of the above indicated NCBI Reference Sequences of the TMIGD2 transcript, and also relates to the corresponding amino acid sequences for example as set forth in SEQ ID NO:14 or in SEQ ID NO:15 or in SEQ ID NO:16, which correspond to the protein sequences defined in NCBI Protein Accession Reference Sequence NP_653216 and in NCBI Protein Accession Reference Sequence NP_001162597 and in NCBI Protein Accession Reference Sequence NP_001295161 encoding the TMIGD2 polypeptide.

The term "TMIGD2" also comprises nucleotide sequences showing a high degree of homology to TMIGD2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO:12 or in SEQ ID NO:13 or amino acid sequences being at least 75%, 80%, 85%, 90%, 91%, 92%, 93%, 94%, 95%, 96%, 97%, 98% or 99% identical to the sequence as set forth in SEQ ID NO: 14 or in SEQ ID NO:15 or in SEQ ID NO:16 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:14 or in SEQ ID NO:15 or in SEQ ID NO:16 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11 or in SEQ ID NO:12 or in SEQ ID NO:13.

The term "VTCN1" refers to the V-Set Domain Containing T Cell Activation Inhibitor 1 gene (Ensembl: ENSG00000134258), for example, to the sequence as defined in NCBI Reference Sequence NM_001253849 or in NCBI Reference Sequence NM_024626 or in NCBI Reference Sequence NM_001253850, specifically, to the nucleotide sequences as set forth in SEQ ID NO:17 or in SEQ ID NO:18 or in SEQ ID NO:19, which correspond to the sequences of the above indicated NCBI Reference Sequences of the VTCN1 transcript, and also relates to the corresponding amino acid sequences for example as set forth in SEQ ID NO:20 or in SEQ ID NO:21 or in SEQ ID NO:22, which correspond to the protein sequences defined in NCBI Protein Accession Reference Sequence NP_001240778 and in NCBI Protein Accession Reference Sequence NP_078902 and in NCBI Protein Accession Reference Sequence NP_001240779.1 encoding the VTCN1 polypeptide.

The term "VTCN1" also comprises nucleotide sequences showing a high degree of homology to VTCN1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:17 or in SEQ ID NO:18 or in SEQ ID NO:19 or amino acid sequences being at least 75%, 80%, 85%, 90%, 91%, 92%, 93%, 94%, 95%, 96%, 97%, 98% or 99% identical to the sequence as set forth in SEQ ID NO:20 or in SEQ ID NO:21 or in SEQ ID NO:22 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or in SEQ ID NO:21 or in SEQ ID NO:22 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:17 or in SEQ ID NO:18 or in SEQ ID NO:19.

The term "biological sample" or "sample obtained from a subject" refers to any biological material obtained via suitable methods known to the person skilled in the art from a subject, e.g., a prostate cancer patient. The biological sample used may be collected in a clinically acceptable manner, e.g., in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "prostate cancer" refers to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 3.0. In another embodiment the term relates to cancer showing PSA levels above 2.0. The term "PSA level" refers to the concentration of PSA in the blood in ng/ml.

The term "non-progressive prostate cancer state" means that a sample of an individual does not show parameter values indicating "biochemical recurrence" and/or "clinical recurrence" and/or "metastases" and/or "castration-resistant disease" and/or "prostate cancer or disease specific death".

The term "progressive prostate cancer state" means that a sample of an individual shows parameter values indicating "biochemical recurrence" and/or "clinical recurrence" and/or "metastases" and/or "castration-resistant disease" and/or "prostate cancer or disease specific death".

The term "biochemical recurrence" generally refers to recurrent biological values of increased PSA indicating the presence of prostate cancer cells in a sample. However, it is also possible to use other markers that can be used in the detection of the presence or that rise suspicion of such presence.

The term "clinical recurrence" refers to the presence of clinical signs indicating the presence of tumour cells as measured, for example using in vivo imaging. The term "metastases" refers to the presence of metastatic disease in organs other than the prostate.

The term "castration-resistant disease" refers to the presence of hormone-insensitive prostate cancer; i.e., a cancer in the prostate that does not any longer respond to androgen deprivation therapy (ADT).

The term "prostate cancer specific death or disease specific death" refers to death of a patient from his prostate cancer.

It is preferred that the two or more immune checkpoint genes comprise three or more, preferably, all of the immune checkpoint genes.

It is preferred that the determining of the prediction of the radiotherapy response comprises combining the gene expression profiles for two or more, for example, 2, 3, 4 or all, of the immune checkpoint genes with a regression function that had been derived from a population of prostate cancer subjects.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t)=H_0(t) \cdot \exp(w_1 \cdot V_1 + w_2 \cdot V_2 + w_3 \cdot V_3 + ...)$, where $V_i, V_2, V_3 ...$ are predictor variables and $H_0(t)$ is the baseline hazard while $H(t)$ is the hazard at any time $t$. The hazard ratio (or the risk to reach the event) is represented by $Ln[H(t)/ H_0(t)]= w_1 \cdot V_1 + w_2 \cdot V_2 + w_3 \cdot V_3 + ...$, where the coefficients or weights $w_1, w_2, w_3 ...$ are estimated by the Cox regression analysis and can be interpreted in a similar manner as for logistic regression analysis.

In one particular realization, the prediction of the radiotherapy response is determined as follows:

$$(w_1 \cdot LAG3) + (w_2 \cdot LGALS9) + (w_3 \cdot TDO2) + (w_4 \cdot TMIGD2) + (w_5 \cdot VTCN1) \qquad (1)$$

where $w_1$ to $w_5$ are weights and LAG3, LGALS9, TDO2, TMIGD22, and VTCN1 are the expression levels of the immune checkpoint genes.

In one example, $w_1$ may be about 0.0 to 1.0, such as 0.5539, $w_2$ may be about -0.5 to 0.5, such as -0.1195, $w_3$ may be about 1.0 to 2.0, such as 1.2637, $w_4$ may be about 0.0 to 1.0, such as 0.2684, and $w_5$ may be about 0.0 to 1.0, such as 0.4617.

The prediction of the radiotherapy response may also be classified or categorized into one of at least two risk groups, based on the value of the prediction of the radiotherapy response. For example, there may be two risk groups, or three risk groups, or four risk groups, or more than four predefined risk groups. Each risk group covers a respective range of (non-overlapping) values of the prediction of the radiotherapy response. For example, a risk group may indicate a probability of occurrence of a specific clinical event from 0 to <0.1 or from 0.1 to <0.25 or from 0.25 to <0.5 or from 0.5 to 1.0 or the like.

It is further preferred that the determining of the prediction of the radiotherapy response is further based on one or more clinical parameters obtained from the subject.

As mentioned above, various measures based on clinical parameters have been investigated. By further basing the prediction of the radiotherapy response on such clinical parameter(s), it can be possible to further improve the prediction.

It is preferred that the one or more clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

It is further preferred that the determining of the prediction of the radiotherapy response comprises combining the gene expression profiles for the two or more immune checkpoint genes and the one or more clinical parameters obtained from the subject with a regression function that had been derived from a population of prostate cancer subjects. In one particular realization, the prediction of the radiotherapy response is determined as follows:

$$(w_6 \cdot \text{IC\_model}) + (w_7 \cdot \text{pGGG}) \tag{2}$$

where $w_6$ and $w_7$ are weights, IC_model is the above-described regression model based on the expression profiles for the two or more, for example, 2, 3, 4 or all, of the immune checkpoint genes, and pGGG is the pathological Gleason grade group. In one example, $w_6$ may be about 0.0 to 1.0, such as 0.665, and $w_7$ may be about 0.5 to 1.5, such as 0.7983.

It is preferred that the biological sample is obtained from the subject before the start of the radiotherapy. The gene expression profiles may be determined in the form of mRNA or protein in tissue of prostate cancer. Alternatively, if the immune checkpoint genes are present in a soluble form, the gene expression profiles may be determined in blood.

It is further preferred that the radiotherapy is radical radiotherapy or salvage radiotherapy.

It is preferred that the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy. The degree to which the prediction is negative may determine the degree to which the recommended therapy deviates from the standard form of radiotherapy.

In a further aspect of the present invention, an apparatus for predicting a response of a prostate cancer subject to radiotherapy is presented, comprising:

- an input adapted to receive data indicative of a gene expression profile for each of two or more, for example, 2, 3, 4 or all, immune checkpoint genes selected from the group consisting of: LAG3, LGALS9, TDO2, TMIGD2, and VTCN1, said gene expression profiles being determined in a biological sample obtained from the subject,
- a processor adapted to determine the prediction of the radiotherapy response based on the gene expression profiles for the two or more immune checkpoint genes, and
- optionally, a providing unit adapted to provide the prediction or a therapy recommendation based on the prediction to a medical caregiver or the subject.

In a further aspect of the present invention, a computer program product is presented comprising instructions which, when the program is executed by a computer, cause the computer to carry out a method comprising:

- receiving data indicative of a gene expression profile for each of two or more, for example, 2, 3, 4 or all, immune checkpoint genes selected from the group consisting of: LAG3, LGALS9, TDO2, TMIGD2, and VTCN1, said gene expression profiles being determined in a biological sample obtained from a prostate cancer subject,
- determining the prediction of the radiotherapy response based on the gene expression profiles for the two or more immune checkpoint genes, and
- optionally, providing the prediction or a therapy recommendation based on the prediction to a medical caregiver or the subject.

In a further aspect of the present invention, a diagnostic kit is presented, comprising:

- at least two primers and/or probes for determining the gene expression profile for each of two or more, for example, 2, 3, 4 or all, immune checkpoint genes selected from the group consisting of: LAG3, LGALS9, TDO2, TMIGD2, and VTCN1, in a biological sample obtained from the subject, and
- an apparatus as defined in claim 10 or a computer program product as defined in claim 11.

In a further aspect of the present invention, a use of the kit as defined in claim 12 is presented.

It is preferred that the use as defined in claim 13 is in a method of predicting a response of a prostate cancer subject to radiotherapy.

In a further aspect of the present invention, a method is presented, comprising:

- receiving a biological sample obtained from a prostate cancer subject,
- using the kit as defined in claim 12 to determine a gene expression profile for each of two or more, for example, 2, 3, 4 or all, immune checkpoint genes selected from the group consisting of: LAG3, LGALS9, TDO2, TMIGD2, and VTCN1, in the biological sample obtained from the subject.

In a further aspect of the present invention, a use of a gene expression profile for each of two or more, for example, 2, 3, 4 or all, immune checkpoint genes selected from the group consisting of: LAG3, LGALS9, TDO2, TMIGD2, and VTCN1, in a method of predicting a response of a prostate cancer subject to radiotherapy is presented, comprising:

- determining, preferably by a processor, the prediction of the radiotherapy response based on the gene expression profiles for the two or more immune checkpoint genes, and
- optionally, providing the prediction or a therapy recommendation based on the prediction to a medical caregiver or the subject.

It shall be understood that the method of claim 1, the diagnostic kit of claim 12, the use of the diagnostic kit of claim 13, the method of claim 15, and the use of the gene expression profiles of claim 16 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a flowchart of an embodiment of a method of predicting a response of a prostate cancer subject to radiotherapy.

Fig. 2 shows a ROC curve analysis of two predictive models.

Fig. 3 shows a Kaplan-Meier curve analysis of the Immune Checkpoint model (IC_model). The clinical endpoint that was tested was the time to metastases (TTM) after the start of salvage radiation therapy (SRT) due to post-surgical disease recurrence.

Fig. 4 shows a Kaplan-Meier curve analysis of the Immune Checkpoint & pGGG combination model (IC&pGGG_model). The clinical endpoint that was tested was the time to metastases (TTM) after the start of salvage radiation therapy (SRT) due to post-surgical disease recurrence.

Fig. 5 shows a Kaplan-Meier curve analysis of the Immune Checkpoint model (IC_model). The clinical endpoint that was tested was the time to prostate cancer specific death (PCa Death) after the start of the salvage radiation therapy (SRT) due to post-surgical disease recurrence.

Fig. 6 shows a Kaplan-Meier curve of the Immune Checkpoint & pGGG combination model (IC&pGGG_model). The clinical endpoint that was tested was the time to prostate cancer specific death (PCa Death) after the start of salvage radiation therapy (SRT) due to post-surgical disease recurrence.

Fig. 7 shows a Kaplan-Meier curve of a Immune Checkpoint 2 gene model (IC_2.1_model). The clinical endpoint that was tested was the time to prostate cancer specific death (PCa Death) after the start of salvage radiation therapy (SRT) due to post-surgical disease recurrence.

Fig. 8 shows a Kaplan-Meier curve of another Immune Checkpoint 2 gene model (IC_2.2_model). The clinical endpoint that was tested was the time to prostate cancer specific death (PCa Death) after the start of salvage radiation therapy (SRT) due to post-surgical disease recurrence.

Fig. 9 shows a Kaplan-Meier curve of another Immune Checkpoint 2 gene model (IC_2.3_model). The clinical endpoint that was tested was the time to prostate cancer specific death (PCa Death) after the start of salvage radiation therapy (SRT) due to post-surgical disease recurrence.

Fig. 10 shows a Kaplan-Meier curve of another Immune Checkpoint 2 gene model (IC_2.4_model). The clinical endpoint that was tested was the time to prostate cancer specific death (PCa Death) after the start of salvage radiation therapy (SRT) due to post-surgical disease recurrence.

Fig. 11 shows a Kaplan-Meier curve of another Immune Checkpoint 2 gene model (IC_2.5_model). The clinical endpoint that was tested was the time to prostate cancer specific death (PCa Death) after the start of salvage radiation therapy (SRT) due to post-surgical disease recurrence.

Fig. 12 shows a Kaplan-Meier curve of another Immune Checkpoint 2 gene model (IC_2.6_model). The clinical endpoint that was tested was the time to prostate cancer specific death (PCa Death) after the start of salvage radiation therapy (SRT) due to post-surgical disease recurrence.

Fig. 13 shows a Kaplan-Meier curve of another Immune Checkpoint 2 gene model (IC_2.7_model). The clinical endpoint that was tested was the time to prostate cancer specific death (PCa Death) after the start of salvage radiation therapy (SRT) due to post-surgical disease recurrence.

Fig. 14 shows a Kaplan-Meier curve of another Immune Checkpoint 2 gene model (IC_2.8_model). The clinical endpoint that was tested was the time to prostate cancer specific death (PCa Death) after the start of salvage radiation therapy (SRT) due to post-surgical disease recurrence.

Fig. 15 shows a Kaplan-Meier curve of a Immune Checkpoint 3 gene model (IC_3. 1 model). The clinical endpoint that was tested was the time to prostate cancer specific death (PCa Death) after the start of salvage radiation therapy (SRT) due to post-surgical disease recurrence.

Fig. 16 shows a Kaplan-Meier curve of another Immune Checkpoint 3 gene model (IC_3.2_model). The clinical endpoint that was tested was the time to prostate cancer specific death (PCa Death) after the start of salvage radiation therapy (SRT) due to post-surgical disease recurrence.

Fig. 17 shows a Kaplan-Meier curve of another Immune Checkpoint 3 gene model (IC_3.3_model). The clinical endpoint that was tested was the time to prostate cancer specific death (PCa Death) after the start of salvage radiation therapy (SRT) due to post-surgical disease recurrence.

Fig. 18 shows a Kaplan-Meier curve of another Immune Checkpoint 3 gene model (IC_3.4_model). The clinical endpoint that was tested was the time to prostate cancer specific death (PCa Death) after the start of salvage radiation therapy (SRT) due to post-surgical disease recurrence.

## DETAILED DESCRIPTION OF EMBODIMENTS

**Overview Of Radiotherapy Response Prediction**

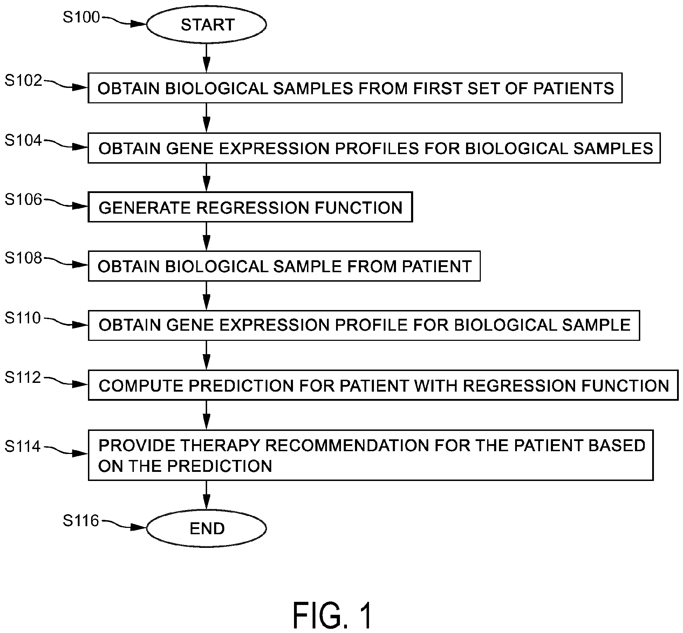

Fig. 1 shows schematically and exemplarily a flowchart of an embodiment of a method of predicting a response of a prostate cancer subject to radiotherapy.

The method begins at step S100.

At step S102, a biological sample is obtained from each of a first set of patients (subjects) diagnosed with prostate cancer. Preferably, monitoring prostate cancer has been performed for these prostate cancer patients over a period of time, such as at least one year, or at least two years, or about five years, after obtaining the biological sample.

At step S104, a gene expression profile for each of two or more, for example, 2, 3, 4 or all, immune checkpoint genes selected from the group consisting of: LAG3, LGALS9, TDO2, TMIGD2, and VTCN1, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the two or more immune checkpoint genes.

At step S106, a regression function for assigning a prediction of the radiotherapy response is determined based on the gene expression profiles for the two or more immune checkpoint genes, LAG3, LGALS9, TDO2, TMIGD2, and/or VTCN1, obtained for at least some of the biological samples obtained for the first set of patients and respective results obtained from the monitoring. In one particular realization, the regression function is determined as specified in Eq. (1) above.

At step S108, a biological sample is obtained from a patient (subject or individual). The patient can be a new patient or one of the first set.

At step S 110, a gene expression profile is obtained for each of the two or more, for example, 2, 3, 4 or all, immune checkpoint genes, e.g., by performing PCR on the biological sample.

At step S 112, a prediction of the radiotherapy response based on the gene expression profiles for the two or more immune checkpoint genes is determined for the patient using the regression function. This will be described in more detail later in the description.

At S114, a therapy recommendation may be provided, e.g., to the patient or his or her guardian, to a doctor, or to another healthcare worker, based on the prediction. To this end, the prediction may be categorized into one of a predefined set of risk groups, based on the value of the prediction. In one particular realization, the prediction of the radiotherapy response may be negative or positive for the effectiveness of the radiotherapy. If the prediction is negative, the recommended therapy may comprise one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

The method ends at S 116.

In one embodiment, the gene expression profiles at steps S 104 and S 110 are determined by detecting mRNA expression using two or more primers and/or probes and/or two or more sets thereof.

In one embodiment, steps S104 and S110 further comprise obtaining one or more clinical parameters from the first set of patients and the patient, respectively. The one or more clinical parameters may comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score. The regression function for assigning the prediction of the radiotherapy response that is determined in step S 106 is then further based on the one or more clinical parameters obtained from at least some of the first set of patients. In step S 112, the prediction of the radiotherapy response is then further based on the one or more clinical parameters, e.g., the pathological Gleason grade group (pGGG), obtained from the patient and is determined for the patient using the regression function.

The immune system interacts in a strong manner with prostate cancer, both on a systemic level and in the tumour microenvironment. Immune checkpoint genes play a central role in the regulation of immune activity. Immune checkpoint genes may therefore provide information on the effectiveness of RT. However, which immune checkpoint genes may have predictive value in this application is extremely difficult to deduce from existing literature due to the many factors that influence the exact function of immune checkpoint genes.

We investigated the extent to which the expression of immune checkpoint genes in prostate cancer tissue correlates with the recurrence of disease after radical RT or SRT.

Of the initial list with immune checkpoint related genes (TABLES 1a and 1b), we have identified five members for which the degree of expression in prostate cancer tissue significantly correlates with mortality after SRT, in a cohort of 151 prostate cancer patients (TABLE 2). In addition, four of the five genes showed a significant correlation with the occurrence of metastases after SRT.

TABLE 1a: Immune checkpoint related genes, expressed on T-Cells, included in analysis.

| Gene | Ensemble | Synonyms |
|------|----------|----------|
| PDCD1 | ENSG00000188389 | CD279, **PD-1**, PD1, SLEB2, Hsle1 |
| CTLA4 | ENSG00000163599 | CD, CD152, CELIAC3, **CTLA-4,** GSE, IDDM12 |
| CD28 | ENSG00000178562 | **CD28** |
| ICOS | ENSG00000163600 | **ICOS,** AILIM, CD278 |
| TMIGD2 | ENSG00000167664 | **TMIGD2,** CD28H, IGPR-1, MGC23244 |
| BTLA | ENSG00000186265 | **BTLA1,** CD272 |
| CD160 | ENSG00000117281 | CD160, BY55, NK1, NK28 |
| TNFRSF9 | ENSG00000049249 | **4-1BB,** CD137, ILA |
| TNFRSF4 | ENSG00000186827 | ACT35, CD134, **OX40,** TXGP1L |
| CD27 | ENSG00000139193 | **CD27,** S152, TNFRSF7, Tp55 |
| CD40LG | ENSG00000102245 | CD154, **CD40L,** HIGM1, IMD3, TNFSF5, TRAP, gp39, hCD40L |
| TNFRSF18 | ENSG00000186891 | AITR, CD357, **GITR** |
| CD96 | ENSG00000153283 | **CD96,** TACTILE |
| CD226 | ENSG00000150637 | CD226, DNAM-1, **DNAM1,** PTA1, TLiSA1 |
| TIGIT | ENSG00000181847 | **TIGIT,** DKFZp667A205, FLJ39873, VSIG9, VSTM3 |
| PVRIG | ENSG00000213413 | **PVRIG,** C7orf15, CD112R, MGC2463 |
| CD200R1 | ENSG00000163606 | **CD200R,** HCRTR2, MOX2R, OX2R |
| CD244 | ENSG00000122223 | **2B4,** NAIL, NKR2B4, Nmrk, SLAMF4, CD244 |
| HAVCR2 | ENSG00000135077 | HAVCR2, CD366, FLJ14428, **TIM3,** TIMD3, Tim-3 |
| ADORA2A | ENSG00000128271 | ADORA2, RDC8 |
| TNFRSF8 | ENSG00000120949 | CD30, D1S166E, KI-1 |
| SIRPA | ENSG00000198053 | SIRP$\alpha$, BIT, CD172a, MFR, MYD-1, P84, PTPNS1, SHPS-1, SHPS1, SIRP, SIRP-ALPHA-1, SIRPalpha, SIRPalpha2 |
| LAG3 | ENSG00000089692 | LAG3, CD223 |

(continued)

| Gene | Ensemble | Synonyms |
|---|---|---|
| CEACAM1 | ENSG00000079385 | CEACAM1, BGP, BGP1, CD66a |

TABLE 1b: Immune checkpoint related genes, expressed on tumour cells and APCs, included in analysis.

| Gene | Ensemble | Synonyms |
|---|---|---|
| CD274 | ENSG00000120217 | B7-H, B7-H1, B7H1, **PD-L1,** PDCD1LG1, PDL1, CD274 |
| PDCD1LG2 | ENSG00000197646 | PDCD1LG2, B7-DC, Btdc, CD273, **PD-L2,** PDL2, bA574F11.2 |
| CD80 | ENSG00000121594 | CD80, B7-1, B7.1, CD28LG, CD28LG1 |
| CD86 | ENSG00000114013 | CD86, B7-2, B7.2, CD28LG2 |
| ICOSLG | ENSG00000160223 | ICOSLG, B7-H2, B7H2, B7RP-1, B7RP1, B7h, CD275, GL50, ICOS-L, **ICOSL,** KIAA0653 |
| CD276 | ENSG00000103855 | CD276, B7-H3, B7H3, B7RP-2 |
| VTCN1 | ENSG00000134258 | VTCN1, B7-H4, B7H4, B7S1, B7x, FLJ22418 |
| VSIR | ENSG00000107738 | VSIR, B7-H5, B7H5, C10orf54, Dies1, GI24, PD-1H, SISP1, **VISTA** |
| HHLA2 | ENSG00000114455 | HHLA2, B7-H5, B7H7, B7y |
| TNFRSF14 | ENSG00000157873 | ATAR, CD270, HVEA, **HVEM,** LIGHTR, TR2, TNFRSF14 |
| TNFSF9 | ENSG00000125657 | CD137L, TNFSF9, 4-1BB-L, 4-1BBL |
| TNFSF4 | ENSG00000117586 | TNFSF4, CD252, **OX-40L,** TXGP1, gp34 |
| CD70 | ENSG00000125726 | **CD70,** CD27L, CD27LG, TNFSF7 |
| CD40 | ENSG00000101017 | **CD40,** Bp50, TNFRSF5, p50 |
| TNFSF18 | ENSG00000120337 | AITRL, TL6, h**GITRL,** TNFSF18 |
| PVR | ENSG00000073008 | PVR, **CD155,** HVED, NECL5, Necl-5, PVS, Tage4 |
| NECTIN2 | ENSG00000130202 | NECTIN2, **CD112,** HVEB, PRR2, PVRL2, PVRR2 |
| CD200 | ENSG00000091972 | CD200, MOX1, MOX2, MRC, OX-2 |
| CD48 | ENSG00000117091 | CD48, BCM1, BLAST, SLAMF2, hCD48, mCD48 |
| LGALS9 | ENSG00000168961 | Galectin-9, GAL9, LGALS9, LGALS9A |
| IDO1 | ENSG00000131203 | IDO1, IDO, INDO |
| TDO2 | ENSG00000151790 | TDO2, **TDO,** TPH2 |
| TNFSF8 | ENSG00000106952 | CD153, CD30LG |
| CD47 | ENSG00000196776 | CD47, IAP, MER6, OA3 |
| CD209 | ENSG00000090659 | DC-SIGN, CDSIGN, CLEC4L, DC-SIGN, DC-SIGN1, CD209 |

Based on the significant correlation with outcome after RT, we expect that the identified molecules will provide predictive value with regard to the effectiveness of radical RT and/or SRT.

TABLE 2: Univariate Cox regression analysis in a cohort with 151 prostate cancer patients. The tested endpoints in the patient cohort were a) post-treatment metastases progression free survival, and b) post-treatment disease specific survival for men undergoing salvage radiation (SRT) after post-surgical disease recurrence. The number of events and percentage per endpoint is indicated in parenthesis. The table indicates for each tested immune checkpoint gene the association in terms of risk (Hazard Ratio) and significance (p-value) to the tested endpoint.

| Data Set | (Prostate RNAseq) | | | |
|---|---|---|---|---|
| # Patients | 151 | | | |
| Outcome | Post-Salvage-Radiation Outcome | | | |
| Endpoint (# events /#patients; % events) | Metastases (#65/#151; 43.0%) | | Prostate Cancer Mortality (#26/#151; 17.2%) | |
| Immune Checkpoint | p-value | HR | p-value | HR |
| LAG3 | 0.02 | 1.5 | 0.02 | 1.7 |
| LGALS9 | 0.002 | 1.6 | 0.05 | 1.6 |
| TDO2 | 0.1 | 2.3 | 0.003 | 4.5 |
| TMIGD2 | 0.04 | 1.6 | 0.03 | 1.7 |
| VTCN1 | 0.007 | 2.1 | 0.03 | 2.1 |

The cell surface molecule Lymphocyte activation gene 3 (LAG3) has diverse biologic effects on T-cell function. It maintains tolerance to self and tumor antigens via direct effects on CD8+ T-cells (see Grosso J.F. et al., "LAG-3 regulates CD8+ T cell accumulation and effector function in murine self- and tumor-tolerance systems", J Clin Invest, Vol. 117, No. 11, pages 3383-3392, 2007, but also on a multitude of other lymphocytes (see Long L. et al., "The promising immune checkpoint LAG-3: From tumor microenvironment to cancer immunotherapy", Genes Cancer, Vol. 9, No. 5-6, pages 176-189, 2018). Increased frequencies of LAG3+ lymphocytes have been described in prostate cancer patients with malignancies (see Norström M.M. et al., "Progression of benign prostatic hyperplasia is associated with pro-inflammatory mediators and chronic activation of prostate-infiltrating lymphocytes", Oncotarget, Vol. 7, No. 17, pages 23581-23593, 2016), and it has been suggested a target for cancer treatment.

Galectin-9, encoded by LGALS9, has been associated with metastasis and immunosuppression. Levels are increased in the endothelium of different tumour types (see Heusschen R. et al., "Endothelial LGALS9 splice variant expression in endothelial cell biology and angiogenesis", Biochim Biophys Acta, Vol. 1842, No. 2, pages 284-292, 2014). One study found increased expression of a family member, galectin 1, during tumour disease progression, which was attributed pro-angiogenic activity in prostate tumours. Contrary, in the same study, expression of Galectin-9 was found to decrease during disease progression (see Laderach D.J. et al., "A unique galectin signature in human prostate cancer progression suggests galectin-1 as a key target for treatment of advanced disease", Cancer Res, Vol. 73, No. 1, pages 86-96, 2013). Galectin-9 induces atypical ubiquitination leading to cell death in PC-3 prostate cancer cells (see Itoh A. et al., "Galectin-9 induces atypical ubiquitination leading to cell death in PC-3 prostate cancer cells", Glycobiology, Vol. 29, No. 1, pages 22-35, 2019). Another study in different cancer types also reported a suggested role for Gal-9 in suppressing metastasis (see Nobumoto A. et al., "Galectin-9 suppresses tumor metastasis by blocking adhesion to endothelium and extracellular matrices", Glycobiology, Vol. 18, No. 9, pages 735-744, 2008). Therefore, explaining the role of galectin-9 in metastasis and cancer-related death in our prostate cancer cohort is not trivial.

IDO1 and TDO2 catalyse the production of the AhR agonist KYN. Activation of AhR by KYN contributes to the immunosuppressive tumour microenvironment and supports cancer immune escape (see Cheong J.E. and Sun L., "Targeting the IDO1/TDO2-KYN-AhR pathway for cancer immunotherapy - Challenges and opportunities", Trends Pharmacol Sci, Vol. 39, No. 3, pages 307-325, 2018). TDO2 expression has been found in multiple human cancers (see Pilotte L. et al., "Reversal of tumoral immune resistance by inhibition of tryptophan 2,3-dioxygenase", Proc Natl Acad Sci USA, Vol. 109, No. 7, pages 2497-2502, 2012). In a study comparing prostate cancer samples from young and older men, TDO2 was one of the genes identified with a higher expression in younger patients, and a role in inflammation-induced immune-suppression was suggested (see Ding Y. et al., "Gene expression differences in prostate cancers between young and old men", PLoS Genet, Vol. 12, No. 12, 2016).

TMIGD2 is widely expressed on the plasma membrane of endothelial and epithelial cells and contributes to cell adhesion and migration (see Rahimi N. et al., "Identification of IGPR-1 as a novel adhesion molecule involved in angiogenesis", Molec Biol Cell, Vol. 23, No. 9, pages 1646-1656, 2012). It is also expressed on lymphocytes. HHLA2, which is constitutively expressed on monocytes and induced on B-cells, was identified to bind TMIGD2, and is able to inhibit proliferation and cytokine production of human CD4 and CD8 T-cells in the presence of TCR signaling. HHLA2/TMIGD2 was suggested as a new pathway of the B7/CD28 families representing a novel immunosuppressive mechanism within the tumor microenvironment and an attractive target for human cancer therapy (see Janakiram M. et al., "HHLA2 and TMIGD2: New immunotherapeutic targets of the B7 and CD28 families", Oncoimmunology, Vol. 4, No. 8, 2015).

VTCN1 encodes a protein belonging to the B7 costimulatory protein family, which are present on the surface of antigen-presenting cells and interact with ligands bound to receptors on the surface of T-cells. The potential clinical significance of VTCN1 is supported by the high expression levels found in numerous tumour tissues, including prostate cancer, and correlation with adverse clinical and pathologic features (see Podojil J.R. and Miller S.D., "Potential targeting of B7-H4 for the treatment of cancer", Immunol Rev, Vol. 276, No. 1, pages 40-51, 2017, and MacGregor H.L. and Ohashi P.S., "Molecular pathways: Evaluating the potential for B7-H4 as an immunoregulatory target", Clin Cancer Res, Vol. 23, No. 12, pages 2934-2941, 2017). VTCN1 may initiate dysfunction of tumour-infiltrating CD8+ T cells and may be targeted for cancer immunotherapy (see Li J. et al., "Co-inhibitory molecule B7 superfamily member 1 expressed by tumor-infiltrating myeloid cells induces dysfunction of anti-tumor CD8+ T cells", Immunity, Vol. 48, No. 4, pages 773-786, 2018).

RESULTS

**Cox Regression Analysis**

We then set out to test whether the combination of these five immune checkpoint genes will exhibit more prognostic value. With Cox regression we modelled the expression levels of the five immune checkpoint genes to prostate cancer specific death after post-surgical salvage RT either with (IC&pGGG_model) or without (IC _model) the presence of the variable pathological Gleason grade group (pGGG). We tested the two models in ROC curve analysis as well as in Kaplan-Meier survival analysis.

The Cox regression functions were derived as follows:

IC_model:

$$(w_1 \cdot LAG3) + (w_2 \cdot LGALS9) + (w_3 \cdot TDO2) + (w_4 \cdot TMIGD2) + (w_5 \cdot VTCN1)$$

IC&pGGG_model:

$$(w_6 \cdot IC\_model) + (w_7 \cdot pGGG)$$

The details for the weights $w_1$ to $w_7$ are shown in the following TABLE 3.

TABLE 3: Variables and weights for the two Cox regression models, i.e., the Immune Checkpoint model (IC _model) and the Immune Checkpoint & pGGG combination model (IC&pGGG_model); NA - not available.

| Variable | Weights | | |
|---|---|---|---|
| Model | | IC_model | IC&pGGG_model |
| LAG3 | $w_1$ | 0.5539 | NA |
| LGALS9 | $w_2$ | -0.1195 | NA |
| TDO2 | $w_3$ | 1.2637 | NA |
| TMIGD2 | $w_4$ | 0.2684 | NA |
| VTCN1 | $w_5$ | 0.4617 | NA |
| IC_model | $w_6$ | NA | 0.665 |
| pGGG | $w_7$ | NA | 0.7983 |

**ROC Curve Analysis**

Next, we tested the combined Cox regression model as outlined above its power to predict 10-year prostate cancer specific death after start of salvage radiation due to post-surgical disease recurrence. The performance of the model was compared to the clinical risk score CAPRA-S (see Cooperberg M.R. et al., "The CAPRA-S score: A straightforward tool for improved prediction of outcomes after radical prostatectomy", Cancer, Vol. 117, No. 22, pages 5039-5046, 2011).

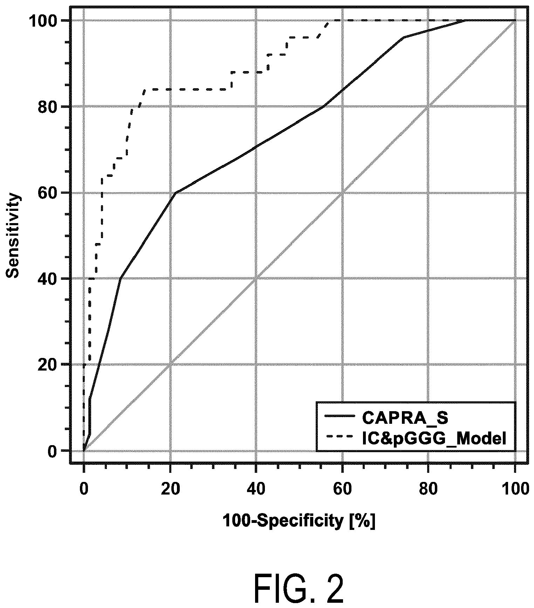

Fig. 2 shows a ROC curve analysis of two predictive models. The IC&pGGG_model (AUC=0.9) is the Cox regression model based on five immune checkpoint genes and the pathology Gleason grade group (pGGG) information. The CAPRA_S (AUC=0.74) is the clinical CAPRA-S score (Cancer of the Prostate Risk Assessment score).

**Kaplan-Meier Survival Analysis**

For Kaplan-Meier curve analysis, the Cox regression function of the two risk models (IC_model and IC&pGGG_model) was categorized into two sub-cohorts based on a cut-off (see description of figures below). The goal was to create patient classes by separating the classes with the mean risk score as calculated from the IC_model and IC&pGGG_model, respectively.

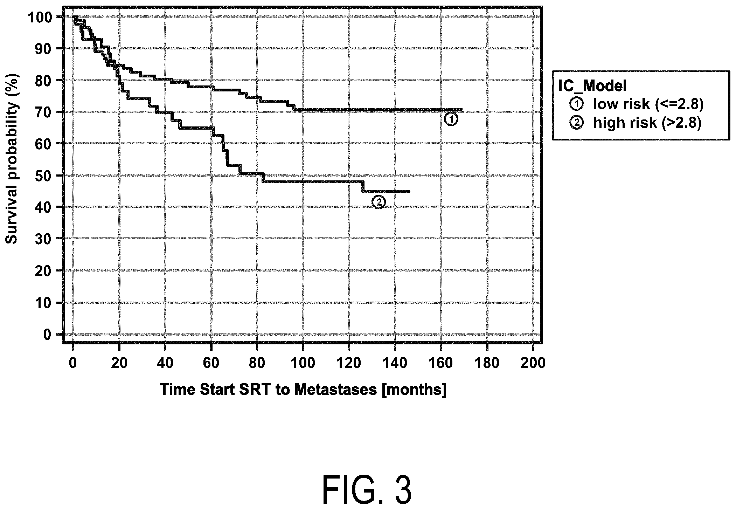

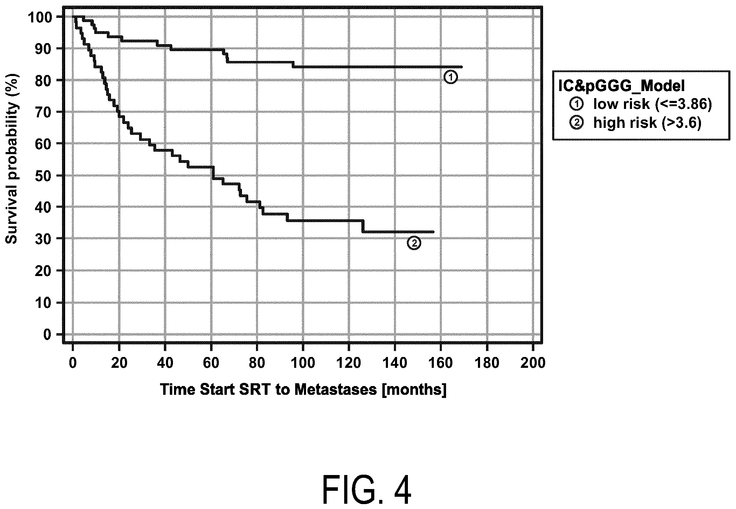

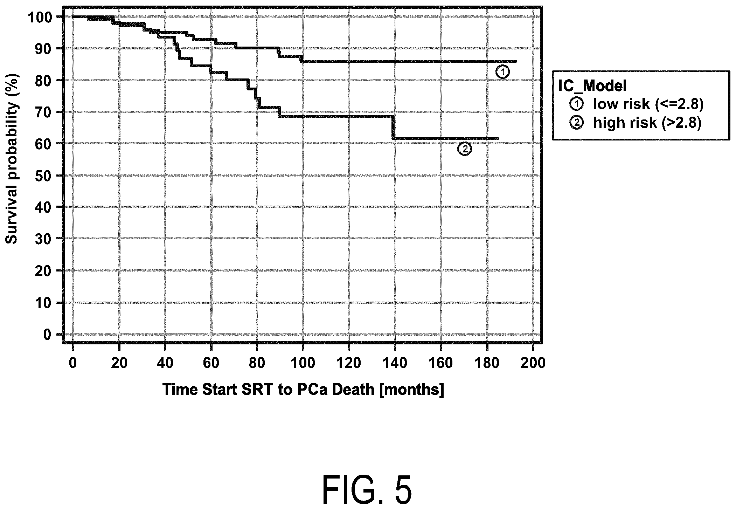

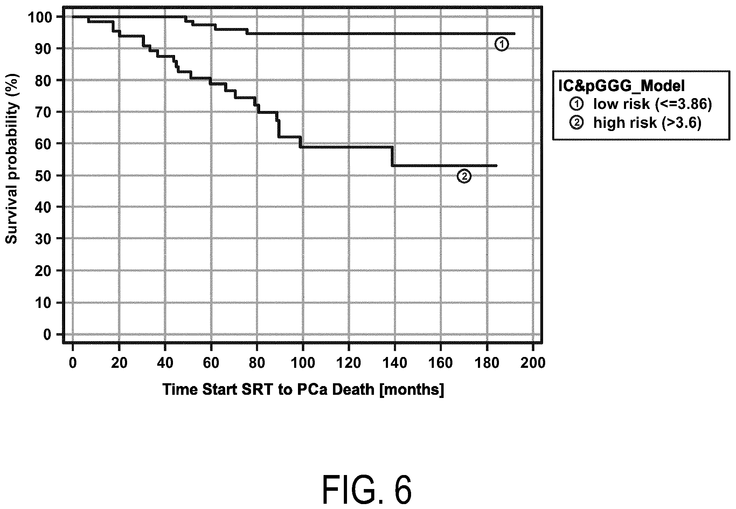

The patient classes represent an increasing risk to experience the tested clinical endpoints of time to development of metastases (Figs. 3 and 4) or time to prostate cancer specific death (Figs. 5 and 6) since the start of salvage RT for the two created risk models (IC_model; IC&pGGG_model).

Fig. 3 shows a Kaplan-Meier curve analysis of the IC_model. The clinical endpoint that was tested was the time to metastases (TTM) after the start of salvage radiation therapy (SRT) due to post-surgical disease recurrence. Patients were stratified into two cohorts (low vs. high) according to their risk to experience the clinical endpoint as predicted by the IC regression model using the value 2.8 as cut-off (logrank p=0.008; HR=2.3; 95% CI=1.2-4.3). The following supplementary lists indicate the number of patients at risk for the IC_model classes analyzed, i.e., the patients at risk at any time interval +20 months after surgery are shown: Low risk: 92, 77, 73, 70, 64, 49, 46, 23, 3, 0; High risk: 43, 34, 30, 28, 20, 18, 17, 6, 0, 0.

Fig. 4 shows a Kaplan-Meier curve analysis of the IC&pGGG_model. The clinical endpoint that was tested was the time to metastases (TTM) after the start of salvage radiation therapy (SRT) due to post-surgical disease recurrence. Patients were stratified into two cohorts (low vs. high) according to their risk to experience the clinical endpoint as predicted by the IC&pGGG regression model using the value 3.86 as cut-off (logrank p<0.0001; HR=6.4; 95% CI=3.6-11.7). The following supplementary lists indicate the number of patients at risk for the IC&pGGG_model classes analyzed, i.e., the patients at risk at any time interval +20 months after surgery are shown: Low risk: 78, 72, 70, 68, 62, 54, 50, 24, 3, 0; High risk: 57, 39, 33, 30, 22, 13, 13, 5, 0, 0.

Fig. 5 shows a Kaplan-Meier curve analysis of the IC_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC regression model using the value 2.8 as cut-off (logrank p=0.01; HR=3.0; 95% CI=1.3-6.9). The following supplementary lists indicate the number of patients at risk for the IC_model classes analyzed, i.e., the patients at risk at any time interval +20 months after surgery are shown: Low risk: 102, 98, 88, 79, 70, 54, 49, 27, 5, 2, 0; High risk: 49, 47, 42, 35, 26, 23, 18, 8, 1, 1, 0.

Fig. 6 shows a Kaplan-Meier curve analysis of the IC&pGGG_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC&pGGG regression model using the value 3.86 as cut-off (logrank p<0.0001; HR=8.4; 95% CI=3.7-18.7). The following supplementary lists indicate the number of patients at risk for the IC&pGGG_model classes analyzed, i.e., the patients at risk at any time interval +20 months after surgery are shown: Low risk: 84, 84, 78, 74, 66, 59, 52, 26, 4, 1, 0; High risk: 67, 61, 52, 40, 30, 18, 15, 9, 2, 2, 0.

For example, the Kaplan-Meier analysis as shown in Figs. 3 to 6 demonstrates the presence of different patient risk groups. The risk group of a patient is determined by the probability to suffer from the respective clinical endpoint (metastases, prostate cancer death) as calculated by the risk model IC_model or IC&pGGG _model. Depending on the predicted risk of a patient (i.e., depending on in which risk group 1 or 2 the patient may belong) different types of interventions might be indicated. In the low risk category, standard of care (SOC) which is SRT potentially combined with SADT (salvage androgen deprivation therapy) delivers acceptable long-term oncological control. Dose escalation of the applied RT and/or combination with chemotherapy might provide improved longitudinal outcomes, and can also be considered. This is definitely not the case for the patient group with a high risk to experience any of the relevant outcomes. In this patient group, escalation of intervention may be indicated. Options for escalation are early combination of SRT (considering higher dose regimens), SADT, and chemotherapy. Other options are alternative therapies like immunotherapies (e.g., Sipuleucil-T), especially since the risk is defined by expression of immune checkpoint molecules, or other experimental therapies.

**Further results**

This section shows additional results for Cox regression models based on only two and three of the identified immune checkpoint genes, respectively. In total, eight different 2 gene models and four different 3 gene models were tested. The details for the weights are shown in the following TABLES 4 and 5, respectively.

TABLE 4: Variables and weights for the 2 gene Cox regression models, i.e., the eight immune checkpoint 2 gene models (IC_2.1_model to IC_2.8_model); NA - not available.

| | IC 2 gene regression models | | | | | | | |
|---|---|---|---|---|---|---|---|---|
| **Variable** | **2.1** | **2.2** | **2.3** | **2.4** | **2.5** | **2.6** | **2.7** | **2.8** |
| **LAG3** | 0.2892 | NA | NA | NA | 0.3647 | NA | NA | 0.3454 |
| **LGALS9** | 0.3237 | 0.3904 | NA | NA | NA | 0.2916 | NA | NA |
| **TDO2** | NA | 0.3727 | 0.231 | NA | 0.3595 | NA | 0.3327 | NA |
| **TMIGD2** | NA | NA | 0.466 | 0.5717 | NA | 0.5461 | NA | NA |
| **VTCN1** | NA | NA | NA | 0.1294 | NA | NA | 0.201 | 0.1829 |

TABLE 5: Variables and weights for the 3 gene Cox regression models, i.e., the four immune checkpoint 3 gene models (IC_3.1_model to IC_3.4_model); NA - not available.

| | IC 3 gene regression models | | | |
|---|---|---|---|---|
| **Variable** | **3.1** | **3.2** | **3.3** | **3.4** |
| **LAG3** | 0.3493 | 0.2868 | 0.342 | NA |
| **LGALS9** | NA | 0.2823 | NA | 0.3261 |
| **TDO2** | 0.3369 | NA | NA | 0.2834 |
| **TMIGD2** | NA | NA | 0.6705 | 0.3489 |
| **VTCN1** | 0.1567 | 0.1317 | 0.1061 | NA |

For Kaplan-Meier curve analysis, the Cox regression function of the twelve risk models (IC_2.1_model to IC_2.8_model and IC_3.1_model to IC_3.4_model) was categorized into two sub-cohorts based on a cut-off (see description of figures below), as described above.

The patient classes represent an increasing risk to experience the tested clinical endpoint of time to prostate cancer specific death since the start of salvage RT for the created risk models.

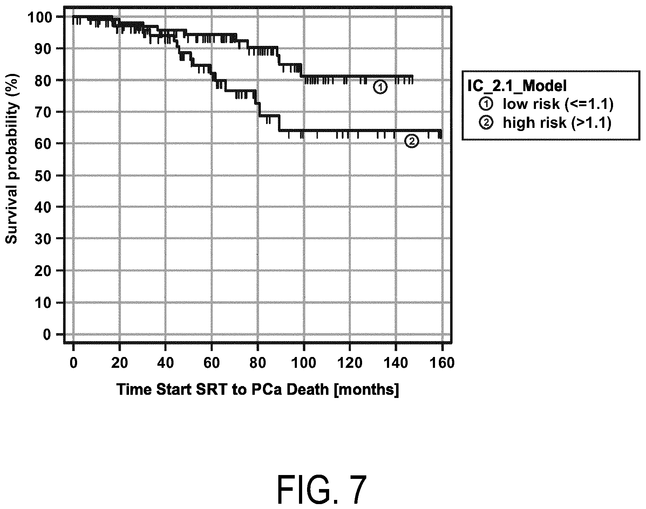

Fig. 7 shows a Kaplan-Meier curve of the IC_2.1_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_2.1_model using the value 1.1 as cut-off (logrank p=0.02; HR=2.6; CI=1.2-5.9). The following supplementary lists indicate the number of patients at risk for the IC_2.1_model classes analyzed, i.e., the patients at risk at any time interval +20 months after surgery are shown: Low risk: 105, 92, 77, 63, 42, 22, 8, 5, 0; High risk: 80, 71, 54, 35, 18, 11, 7, 3, 0.

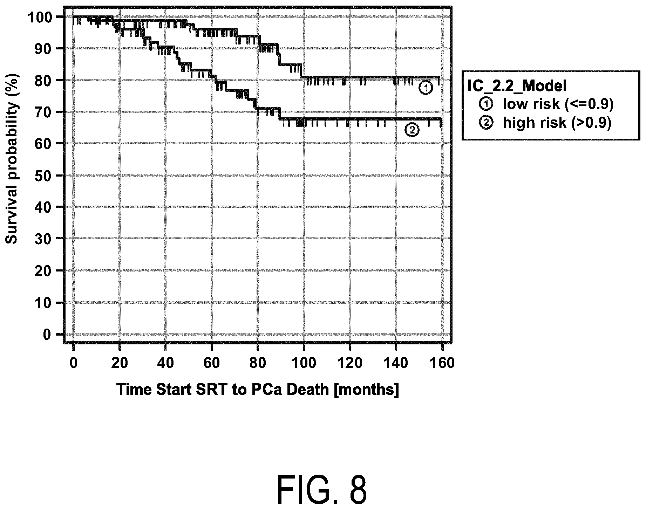

Fig. 8 shows a Kaplan-Meier curve of the IC_2.2_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_2.2_model using the value 0.9 as cut-off (logrank p=0.009; HR=2.9; CI=1.3-6.4). The following supplementary lists indicate the number of patients at risk for the IC_2.2_model classes analyzed, i.e., the patients at risk at any time interval +20 months after surgery are shown: Low risk: 96, 89, 78, 59, 36, 20, 9, 6, 0; High risk: 89, 74, 53, 39, 24, 13, 6, 2, 0.

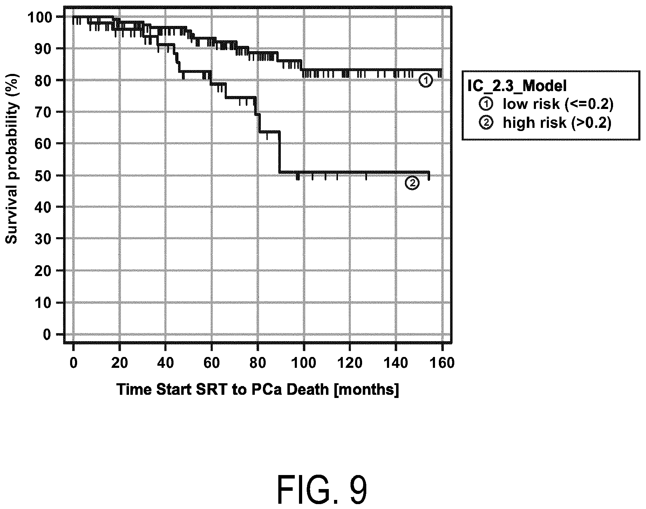

Fig. 9 shows a Kaplan-Meier curve of the IC_2.3_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_2.3_model using the value 0.2 as cut-off (logrank p=0.001; HR=4.6; CI=1.8-11.5). The following supplementary lists indicate the number of patients at risk for the IC_2.3_model classes analyzed, i.e., the patients at risk at any time interval +20 months after surgery are shown: Low risk: 131, 117, 96, 78, 47, 28, 13, 7, 0; High risk: 54, 46, 35, 20, 13, 5, 2, 1, 0.

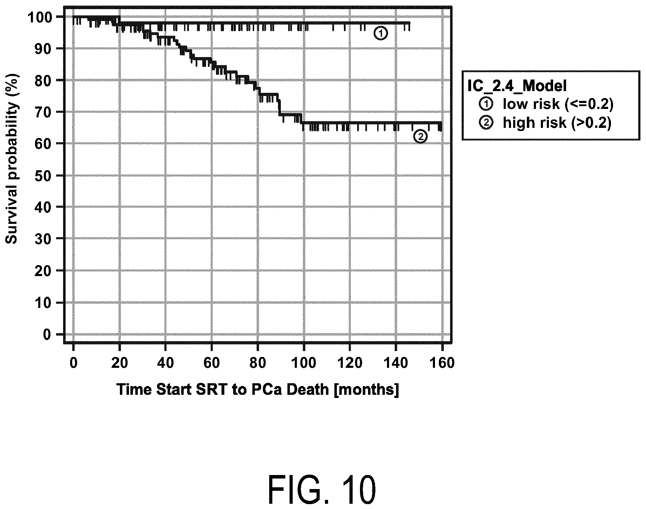

Fig. 10 shows a Kaplan-Meier curve of the IC_2.4_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_2.4_model using the value 0.2 as cut-off (logrank p=0.02; HR=2.7; CI=1.2-6.1). The following supplementary lists indicate the number of patients at risk for the IC_2.4_model classes analyzed, i.e., the patients at risk at any time interval +20 months after surgery are shown: Low risk: 61, 53, 41, 33, 18, 8, 4, 2, 0; High risk: 124, 110, 90, 65, 42, 25, 11, 6, 0.

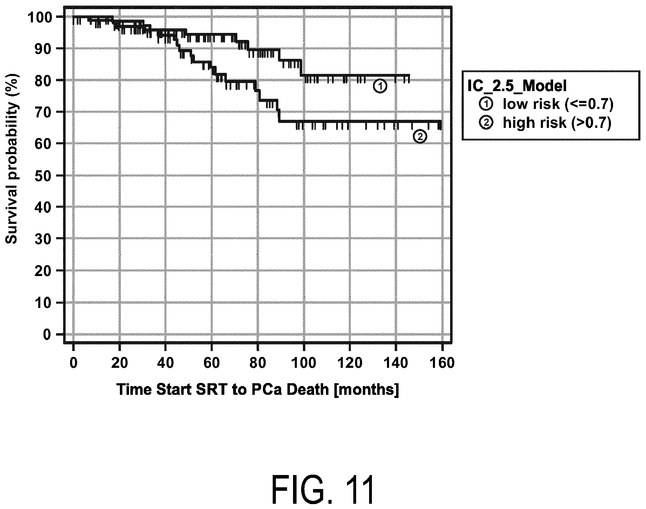

Fig. 11 shows a Kaplan-Meier curve of the IC_2.5_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_2.5_model using the value 0.7 as cut-off (logrank p=0.05; HR=2.2; CI=1.0-4.9). The following supplementary lists indicate the number of patients at risk for the IC_2.5_model classes analyzed, i.e., the patients at risk at any time interval +20 months after surgery are shown: Low risk: 100, 90, 72, 55, 34, 17, 6, 3, 0; High risk: 85, 73, 59, 43, 26, 16, 9, 5, 0.

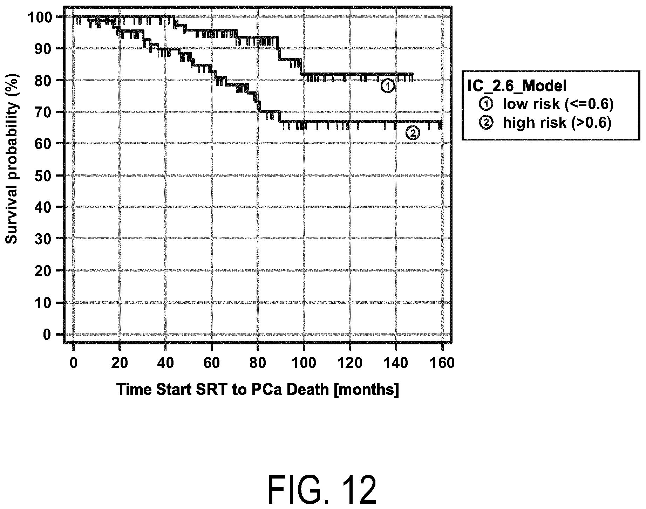

Fig. 12 shows a Kaplan-Meier curve of the IC_2.6_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_2.6_model using the value 0.6 as cut-off (logrank p=0.008; HR=2.9; CI=1.3-6.4). The following supplementary lists indicate the number of patients at risk for the IC_2.6_model classes analyzed, i.e., the patients at risk at any time interval +20 months after surgery are shown: Low risk: 94, 83, 72, 56, 34, 19, 8, 4, 0; High risk: 91, 80, 59, 42, 26, 14, 7, 4, 0.

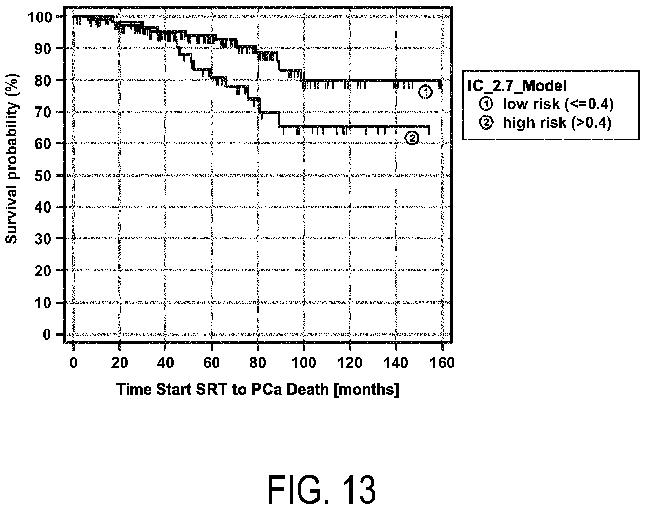

Fig. 13 shows a Kaplan-Meier curve of the IC_2.7_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_2.7_model using the value 0.4 as cut-off (logrank p=0.05; HR=2.3; CI=1.0-5.2). The following supplementary lists indicate the number of patients at risk for the IC_2.7_model classes analyzed, i.e., the patients at risk at any time interval +20 months after surgery are shown: Low risk: 118, 105, 84, 66, 42, 22, 11, 7, 0; High risk: 67, 58, 47, 32, 18, 11, 4, 1, 0.

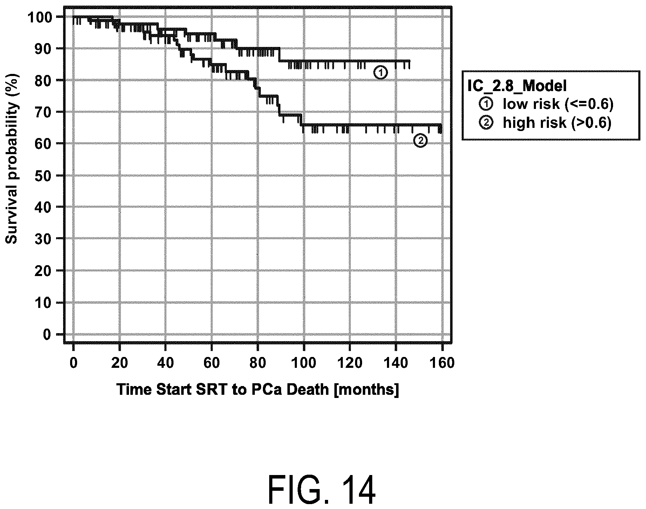

Fig. 14 shows a Kaplan-Meier curve of the IC_2.8_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_2.8_model using the value 0.6 as cut-off (logrank p=0.04; HR=2.2; CI=1.0-4.9). The following supplementary lists indicate the number of patients at risk for the IC_2.8_model classes analyzed, i.e., the patients at risk at any time interval +20 months after surgery are shown: Low risk: 90, 77, 61, 49, 30, 14, 6, 3, 0; High risk: 95, 86, 70, 49, 30, 19, 9, 5, 0.

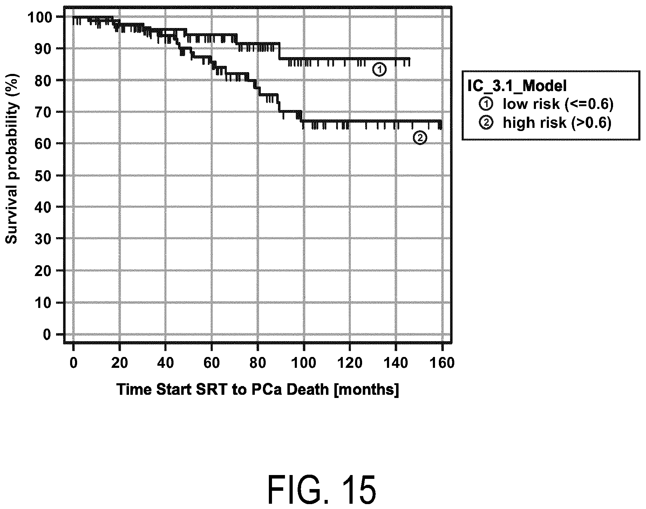

Fig. 15 shows a Kaplan-Meier curve of the IC_3.1_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_3.1_model using the value 0.6 as cut-off (logrank p=0.04; HR=2.3; CI=1.0-5.0). The following supplementary lists indicate the number of patients at risk for the IC_3.1_model classes analyzed, i.e., the patients at risk at any time interval +20 months after surgery are shown: Low risk: 81, 73, 58, 45, 27, 13, 6, 3, 0; High risk: 104, 90, 73, 53, 33, 20, 9, 5, 0.

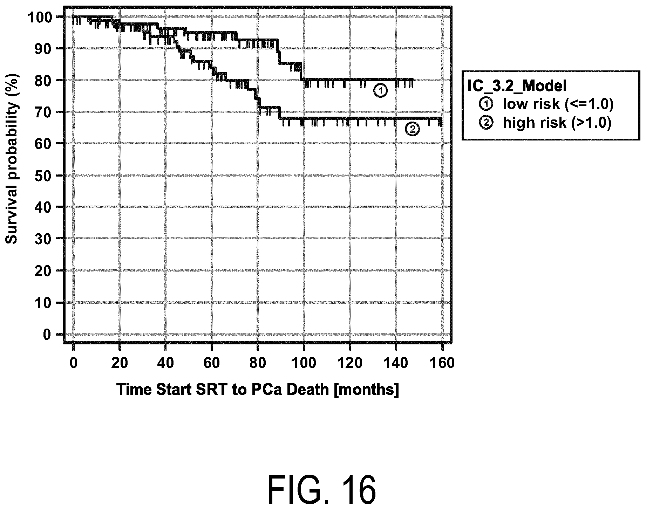

Fig. 16 shows a Kaplan-Meier curve of the IC_3.2_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_3.2_model using the value 1.0 as cut-off (logrank p=0.04; HR=2.3; CI=1.0-5.0). The following supplementary lists indicate the number of patients at risk for the IC_3.2_model classes analyzed, i.e., the patients at risk at any time interval +20 months after surgery are shown: Low risk: 93, 80, 67, 53, 34, 16, 7, 5, 0; High risk: 92, 83, 64, 45, 26, 17, 8, 3, 0.

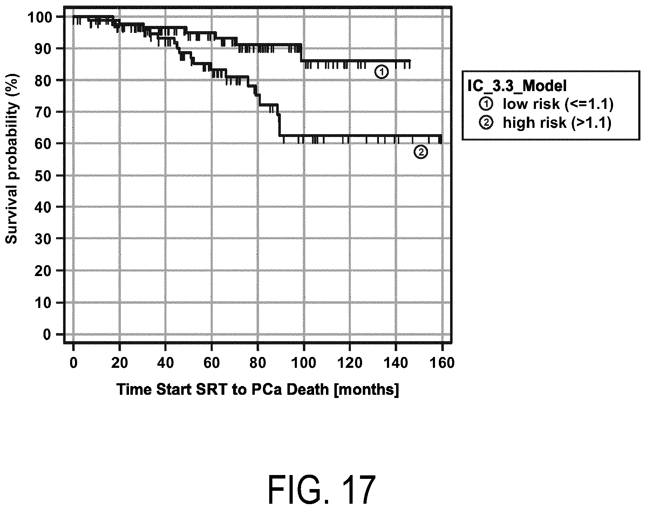

Fig. 17 shows a Kaplan-Meier curve of the IC_3.3_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_3.3_model using the value 0.7 as cut-off (logrank p=0.009; HR=2.9; CI=1.3-6.3). The following supplementary lists indicate the number of patients at risk for the IC_3.3_model classes analyzed, i.e., the patients at risk at any time interval +20 months after surgery are shown: Low risk: 100, 87, 69, 55, 34, 17, 6, 3, 0; High risk: 85, 76, 62, 43, 26, 16, 9, 5, 0.

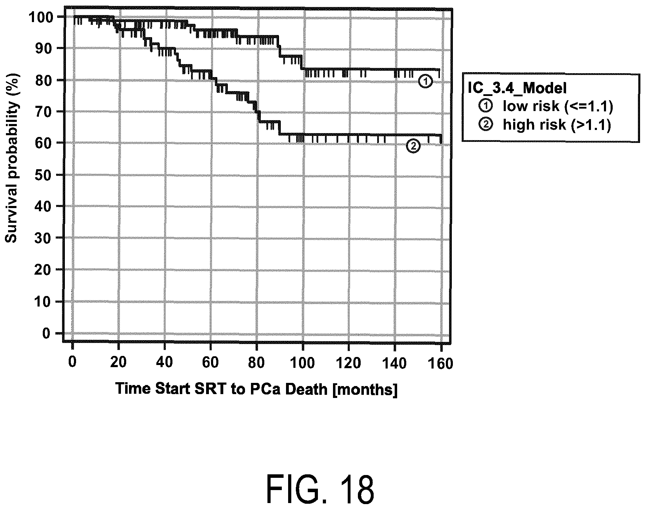

Fig. 18 shows a Kaplan-Meier curve of the IC_3.4_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IC_3.4_model using the value 0.8 as cut-off (logrank p=0.001; HR=3.7; CI=1.7-8.2). The following supplementary lists indicate the number of patients at risk for the IC_3.4_model classes analyzed, i.e., the patients at risk at any time interval +20 months after surgery are shown: Low risk: 98, 91, 78, 60, 37, 22, 9, 6, 0; High risk: 87, 72, 53, 38, 23, 11, 6, 2, 0.

The Kaplan-Meier analysis as shown in Figs. 7 to 18 demonstrates that different patient risk groups can also be distinguished using risk models that are only based on a subset of the identified immune checkpoint genes, for example, two or three of the genes.

## Discussion

The effectiveness of both radical RT and SRT for localized prostate cancer is limited, resulting in disease progression and ultimately death of patients, especially for those at high risk of recurrence. The prediction of the therapy outcome is very complicated as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the response to radical RT and to SRT, in order to increase the success rate of these therapies.

We have identified molecules of which expression shows a significant relation to mortality after radical RT and SRT and therefore are expected to improve the prediction of the effectiveness of these treatments. An improved prediction of effectiveness of RT for each patient be it in the radical or the salvage setting, will improve therapy selection and potentially survival. This can be achieved by 1) optimizing RT for those patients where RT is predicted to be effective (e.g. by dose escalation or a different starting time) and 2) guiding patients where RT is predicted not to be effective to an alternative, potentially more effective form of treatment. Further, this would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The invention relates to a method of predicting a response of a prostate cancer subject to radiotherapy, comprising determining or receiving the result of a determination of a gene expression profile for each of two or more, for example, 2, 3, 4 or all, immune checkpoint genes selected from the group consisting of: LAG3, LGALS9, TDO2, TMIGD2, and VTCN1, said gene expression profiles being determined in a biological sample obtained from the subject, determining, preferably by a processor, the prediction of the radiotherapy response based on the gene expression profiles for the two or more immune checkpoint genes, and optionally, providing the prediction or a therapy recommendation based on the prediction to a medical caregiver or the subject. Since the status of the immune system and of the immune microenvironment have an impact on therapy effectiveness, the ability to identify markers predictive for this effect might help to be better able to predict overall RT response. Immune checkpoint genes play a central role in the regulation of immune activity. The identified immune checkpoint genes were found to exhibit a significant correlation with outcome after RT, wherefore we expect that they will provide predictive value with regard to the effectiveness of radical RT and/or SRT.

The attached Sequence Listing, entitled 2020PF00064_Sequence Listing_ST25 is incorporated herein by reference, in its entirety.

**Claims**

1. A method of predicting a response of a prostate cancer subject to radiotherapy, comprising:

   - determining a gene expression profile for each of two or more, for example, 2, 3, 4 or all, immune checkpoint genes selected from the group consisting of: LAG3, LGALS9, TDO2, TMIGD2, and VTCN1, said gene expression profiles being determined in a biological sample obtained from the subject,
   - determining, the prediction of the radiotherapy response based on the gene expression profiles for the two or more immune checkpoint genes, and
   - optionally, providing the prediction or a therapy recommendation based on the prediction to a medical caregiver or the subject.

2. A method of predicting a response of a prostate cancer subject to radiotherapy, comprising:

   - receiving the result of a determination of a gene expression profile for each of two or more, for example, 2, 3, 4 or all, immune checkpoint genes selected from the group consisting of: LAG3, LGALS9, TDO2, TMIGD2, and VTCN1, said gene expression profiles being determined in a biological sample obtained from the subject,
   - determining, by a processor, the prediction of the radiotherapy response based on the gene expression profiles for the two or more immune checkpoint genes, and
   - optionally, providing the prediction or a therapy recommendation based on the prediction to a medical caregiver or the subject.

3. The method as defined in claim 1, wherein the two or more immune checkpoint genes comprise three or more, preferably, all of the immune checkpoint genes.

4. The method as defined in any of claims -3, wherein the determining of the prediction of the radiotherapy response comprises combining the gene expression profiles for two or more, for example, 2, 3, 4 or all, of the immune checkpoint genes with a regression function that had been derived from a population of prostate cancer subjects.

5. The method as defined in any of claims 1-4, wherein the determining of the prediction of the radiotherapy response is further based on one or more clinical parameters obtained from the subject.

6. The method as defined in claim 5, wherein the clinical parameters comprise one or more of: (i) a prostate-specific antigen (PSA) level; (ii) a pathologic Gleason score (pGS); iii) a clinical tumour stage; iv) a pathological Gleason grade group (pGGG); v) a pathological stage; vi) one or more pathological variables, for example, a status of surgical margins and/or a lymph node invasion and/or an extra-prostatic growth and/or a seminal vesicle invasion; vii) CAPRA-S; and viii) another clinical risk score.

7. The method as defined in claim 5 or 6, wherein the determining of the prediction of the radiotherapy response comprises combining the gene expression profiles for the two or more immune checkpoint genes and the one or more clinical parameters obtained from the subject are combined with a regression function that had been derived from a population of prostate cancer subjects.

8. The method as defined in any of claims 1 to 7, wherein the biological sample is obtained from the subject before the start of the radiotherapy.

9. The method as defined in any of claims 1 to 8, wherein the radiotherapy is radical radiotherapy or salvage radiotherapy.

10. The method as defined in any of claims 1 to 9, wherein the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

11. An apparatus for predicting a response of a prostate cancer subject to radiotherapy, comprising:

- an input adapted to receive data indicative of a gene expression profile for each of two or more, for example, 2, 3, 4 or all, immune checkpoint genes selected from the group consisting of: LAG3, LGALS9, TDO2, TMIGD2, and VTCN1, said gene expression profiles being determined in a biological sample obtained from the subject,
- a processor adapted to determine the prediction of the radiotherapy response based on the gene expression profiles for the two or more immune checkpoint genes, and
- optionally, a providing unit adapted to provide the prediction or a therapy recommendation based on the prediction to a medical caregiver or the subject.

12. A computer program product comprising instructions which, when the program is executed by a computer, cause the computer to carry out a method comprising:

- receiving data indicative of a gene expression profile for each of two or more, for example, 2, 3, 4 or all, immune checkpoint genes selected from the group consisting of: LAG3, LGALS9, TDO2, TMIGD2, and VTCN1, said gene expression profiles being determined in a biological sample obtained from a prostate cancer subject,
- determining the prediction of the radiotherapy response based on the gene expression profiles for the two or more immune checkpoint genes, and
- optionally, providing the prediction or a therapy recommendation based on the prediction to a medical caregiver or the subject.

13. A diagnostic kit, comprising:

- at least two primers and/or probes for determining the gene expression profile for each of two or more, for example, 2, 3, 4 or all, immune checkpoint genes selected from the group consisting of: LAG3, LGALS9, TDO2, TMIGD2, and VTCN1, in a biological sample obtained from the subject, and
- an apparatus as defined in claim 11 or a computer program product as defined in claim 12.

14. Use of the kit as defined in claim 13.

15. The use of the kit as defined in claim 14 in a method of predicting a response of a prostate cancer subject to radiotherapy.

16. A method, comprising:

- receiving a biological sample obtained from a prostate cancer subject,
- using the kit as defined in claim 11 to determine a gene expression profile for each of two or more, for example, 2, 3, 4 or all, immune checkpoint genes selected from the group consisting of: LAG3, LGALS9, TDO2, TMIGD2, and VTCN1, in the biological sample obtained from the subject.

17. Use of a gene expression profile for each of two or more, for example, 2, 3, 4 or all, immune checkpoint genes selected from the group consisting of: LAG3, LGALS9, TDO2, TMIGD2, and VTCN1, in a method of predicting a response of a prostate cancer subject to radiotherapy, comprising:

- determining, by a processor, the prediction of the radiotherapy response based on the gene expression profiles for the two or more immune checkpoint genes, and

- optionally, providing the prediction or a therapy recommendation based on the prediction to a medical caregiver or the subject.

**Patentansprüche**

1. Ein Verfahren zur Vorhersage des Ansprechens eines Patienten mit Prostatakarzinom auf Strahlentherapie, umfassend:

    - Bestimmung eines Genexpressionsprofils für jedes von zwei oder mehr, z.B. 2, 3, 4 oder allen, Immuncheckpoint-Genen, ausgewählt aus der Gruppe bestehend aus: LAG3, LGALS9, TDO2, TMIGD2 und VTCN1, die besagten Genexpressionsprofile werden in einer biologischen Probe bestimmt, die vom Patienten gewonnen wird,
    - Bestimmung der Vorhersage der Ansprechens auf Strahlentherapie auf Grundlage der Genexpressionsprofile für die zwei oder mehr Immuncheckpoint-Gene und
    - wahlweise Bereitstellung der Prognose oder einer Therapieempfehlung auf Grundlage der Prognose an eine medizinische Betreuungsperson oder den Patienten.

2. Ein Verfahren zur Vorhersage des Ansprechens eines Patienten mit Prostatakarzinom auf Strahlentherapie, umfassend:

    - Empfangen des Ergebnisses einer Bestimmung eines Genexpressionsprofils für jedes von zwei oder mehr, z.B. 2, 3, 4 oder allen, Immuncheckpoint-Genen, ausgewählt aus der Gruppe bestehend aus: LAG3, LGALS9, TDO2, TMIGD2 und VTCN1, die Genexpressionsprofile werden in einer biologischen Probe bestimmt, die vom Patienten gewonnen wird,
    - Bestimmung, durch einen Prozessor, der Vorhersage der Ansprechens auf Strahlentherapie auf der Grundlage der Genexpressionsprofile für die zwei oder mehr Immuncheckpoint-Gene und
    - wahlweise Bereitstellung der Prognose oder einer Therapieempfehlung auf der Grundlage der Prognose an eine medizinische Betreuungsperson oder den Patienten.

3. Verfahren nach Anspruch 1, wobei die zwei oder mehr Immun-Checkpoint-Gene drei oder mehr, vorzugsweise alle Immun-Checkpoint-Gene umfassen.

4. Verfahren nach einem der Ansprüche 1-3, wobei die Bestimmung der Vorhersage der Ansprechens auf Strahlentherapie die Kombination der Genexpressionsprofile für zwei oder mehr, z.B. 2, 3, 4 oder alle Immun-Checkpoint-Gene umfasst, mit einer Regressionsfunktion, die aus einer Population von Prostatakrebspatienten abgeleitet worden war.

5. Verfahren nach einem der Ansprüche 1-4, wobei die Bestimmung der Vorhersage der Ansprechens auf Strahlentherapie weiter auf einem oder mehreren klinischen Parametern beruht, die von dem Patienten erhalten werden.

6. Verfahren nach Anspruch 5, wobei die klinischen Parameter einen oder mehrere der folgenden umfassen: (i) Einen prostataspezifischen Antigen-(PSA-)Spiegel; (ii) einen pathologischen Gleason-Score (pGS); iii) ein klinisches Tumorstadium; iv) eine pathologische Gleason-Gradgruppe (pGGG); v) ein pathologisches Stadium; vi) eine oder mehrere pathologische Variablen, z.B. ein Status der chirurgischen Ränder und/oder einer Lymphknoteninvasion und/oder eines extraprostatischen Wachstums und/oder einer Samenblaseninvasion; vii) CAPRA-S und viii) ein anderer klinischer Risikoscore.

7. Verfahren nach Anspruch 5 oder 6, wobei die Bestimmung der Vorhersage der Ansprechens auf Strahlentherapie die Kombination der Genexpressionsprofile für die zwei oder mehr Immun-Checkpoint-Gene umfasst und wo die ein oder mehrere klinischen Parameter, die vom Patienten erhalten wurden, mit einer Regressionsfunktion kombiniert werden, die aus einer Population von Prostatakrebspatienten abgeleitet wurde.

8. Verfahren nach einem der Ansprüche 1 bis 7, wobei die biologische Probe vor Beginn der Strahlentherapie vom Patienten gewonnen wird.

9. Verfahren nach einem der Ansprüche 1 bis 8, **dadurch gekennzeichnet, dass** die Strahlentherapie eine radikale Strahlentherapie oder eine erhaltende Strahlentherapie ist.

10. Verfahren nach einem der Ansprüche 1 bis 9, wobei die Vorhersage der Ansprechens auf Strahlentherapie negativ oder positiv für die Wirksamkeit der Strahlentherapie ist, wobei eine Therapie auf der Grundlage der Vorhersage empfohlen wird und, wenn die Vorhersage negativ ist, die empfohlene Therapie eine oder mehrere der folgenden Optionen umfasst: (i) Strahlentherapie, die früher als gewöhnlich durchgeführt wird; (ii) Strahlentherapie mit einer erhöhten Strahlendosis; (iii) eine adjuvante Therapie, wie z.B. eine Androgendeprivationstherapie; und iv) eine alternative Therapie, bei der es sich nicht um eine Strahlentherapie handelt.

11. Gerät zur Vorhersage des Ansprechens eines Patienten mit Prostatakarzinom auf Strahlentherapie, umfassend:

- Eine Eingabe, die so angepasst ist, dass Daten empfangen werden, die auf ein Genexpressionsprofil für jedes von zwei oder mehr, z.B. 2, 3, 4 oder alle, hinweisen; Immuncheckpoint-Gene, ausgewählt aus der Gruppe bestehend aus LAG3, LGALS9, TDO2, TMIGD2 und VTCN1, die Genexpressionsprofile werden in einer biologischen Probe bestimmt, die vom Patienten gewonnen wird,
- ein Prozessor, der die Vorhersage der Ansprechens auf Strahlentherapie auf Grundlage der Genexpressionsprofile für die zwei oder mehr Immuncheckpoint-Gene bestimmt, und
- optional eine Bereitstellungseinheit, die auf Grundlage der Vorhersage für eine medizinische Betreuungsperson oder den Patienten die Vorhersage oder Therapieempfehlung liefert.

12. Ein Computerprogrammprodukt, das Anweisungen enthält, die bei Ausführung des Programms durch einen Computer bewirken, dass der Computer ein Verfahren durchführt, das Folgendes umfasst:

- Erhalten von Daten, die auf ein Genexpressionsprofil für jedes von zwei oder mehr, z.B. 2, 3, 4 oder allen, Immuncheckpoint-Genen hinweisen, ausgewählt aus der Gruppe bestehend aus: LAG3, LGALS9, TDO2, TMIGD2 und VTCN1, die Genexpressionsprofile werden in einer biologischen Probe bestimmt, die von einem Prostatakrebspatienten gewonnen wurde,
- Bestimmung der Vorhersage der Ansprechens auf Strahlentherapie auf Grundlage der Genexpressionsprofile für die zwei oder mehr Immuncheckpoint-Gene und
- wahlweise Bereitstellung der Prognose oder einer Therapieempfehlung auf der Grundlage der Prognose an eine medizinische Betreuungsperson oder den Patienten.

13. Ein Diagnose-Kit, umfassend:

- Mindestens zwei Primer und/oder Sonden zur Bestimmung des Genexpressionsprofils für jedes von zwei oder mehr, z.B. 2, 3, 4 oder alle, Immuncheckpoint-Gene, ausgewählt aus der Gruppe bestehend aus: LAG3, LGALS9, TDO2, TMIGD2 und VTCN1, in einer biologischen Probe, die vom Patienten gewonnen wurde, und
- ein Gerät gemäß Anspruch 11 oder ein Computerprogrammprodukt gemäß Anspruch 12.

14. Verwendung des Kits gemäß Anspruch 13.

15. Verwendung des Kits gemäß Anspruch 14 in einem Verfahren zur Vorhersage des Ansprechens eines Patienten mit Prostatakarzinom auf Strahlentherapie.

16. Ein Verfahren, das Folgendes umfasst:

- Erhalten einer biologischen Probe von einem Prostatakrebspatienten,
- Verwendung des Kits nach Anspruch 11 zur Bestimmung eines Genexpressionsprofils für jedes von zwei oder mehr, z.B. 2, 3, 4 oder allen, Immuncheckpoint-Genen, ausgewählt aus der Gruppe bestehend aus: LAG3, LGALS9, TDO2, TMIGD2 und VTCN1, in der biologischen Probe, die vom Patienten gewonnen wurde.

17. Verwendung eines Genexpressionsprofils für jedes von zwei oder mehr, z.B. 2, 3, 4 oder allen, Immuncheckpoint-Genen, ausgewählt aus der Gruppe bestehend aus: LAG3, LGALS9, TDO2, TMIGD2 und VTCN1, in einem Verfahren zur Vorhersage des Ansprechens eines Patienten mit Prostatakarzinom auf Strahlentherapie, umfassend:

- Bestimmung, durch einen Prozessor, der Vorhersage der Ansprechens auf Strahlentherapie auf Grundlage der Genexpressionsprofile für die zwei oder mehr Immuncheckpoint-Gene und
- wahlweise Bereitstellung der Prognose oder einer Therapieempfehlung auf Grundlage der Prognose an eine medizinische Betreuungsperson oder den Patienten.

**Revendications**

1. Le procédé de prédiction d'une réponse d'un cancer de la prostate soumis à une radiothérapie comprend:

   - la détermination d'un profil d'expression génétique pour chacun de deux ou plusieurs, par exemple 2, 3, 4 ou tous, gènes de points de contrôle immuns sélectionnés dans le groupe constitué de: LAG3, LGALS9, TDO2, TMIGD2 et VTCN1, lesdits profils d'expression génétiques étant déterminés dans un échantillon biologique prélevé sur le sujet,
   - déterminer, la prédiction de la réponse à la radiothérapie basée sur les profils d'expression génique pour les deux gènes ou plus de points de contrôle immunitaire, et
   - facultativement, fournir la prédiction ou une recommandation thérapeutique basée sur la prédiction à un soignant ou au sujet.

2. Le procédé de prédiction d'une réponse d'un cancer de la prostate soumis à une radiothérapie comprend:

   - la réception du résultat d'une détermination d'un profil d'expression génique pour chacun de deux ou plusieurs, par exemple 2, 3, 4 ou tous, gènes de points de contrôle immunitaires sélectionnés dans le groupe constitué de: LAG3, LGALS9, TDO2, TMIGD2 et VTCN1, lesdits profils d'expression génique étant déterminés dans un échantillon biologique prélevé sur le sujet,
   - déterminer, par un processeur, la prédiction de la réponse à la radiothérapie sur la base des profils d'expression des gènes pour les deux gènes ou plus du point de contrôle immunitaire, et
   - facultativement, fournir la prédiction ou une recommandation thérapeutique basée sur la prédiction à un soignant médical ou au sujet.

3. Le procédé selon la revendication 1, dans lequel les deux ou plusieurs gènes de points de contrôle immuns comprennent trois ou plus, de préférence tous les gènes de points de contrôle immuns.

4. Le procédé selon l'une quelconque des revendications 1-3, dans lequel la détermination de la prédiction de la réponse de radiothérapie comprend la combinaison des profils d'expression génique pour deux ou plusieurs, par exemple 2, 3, 4 ou tous, des gènes de point de contrôle immunitaire avec une fonction de régression qui avaient été dérivés d'une population de sujets atteints du cancer de la prostate.

5. Le procédé selon l'une quelconque des revendications 1-4, dans lequel la détermination de la prédiction de la réponse de radiothérapie est en outre basée sur un ou plusieurs paramètres cliniques obtenus à partir du sujet.

6. Le procédé selon la revendication 5, dans lequel les paramètres cliniques comprennent un ou plusieurs des éléments suivants: (i) un niveau d'antigène prostatique spécifique (PSA); (ii) un score de Gleason pathologique (pGS); iii) un stade clinique de la tumeur; iv) un groupe de grade de Gleason pathologique (pGGG); v) un stade pathologique; vi) une ou plusieurs variables pathologiques, par exemple, un état des marges chirurgicales et/ou une invasion ganglionnaire et/ou une croissance extra prostatique et/ou une invasion vésiculaire séminale; vii) CAPRA-S; et viii) un autre score de risque clinique.

7. Le procédé selon la revendication 5 ou 6, la détermination de la prédiction de la réponse de radiothérapie comprend la combinaison des profils d'expression génique pour les deux ou plusieurs gènes de point de contrôle immunitaire et le ou les paramètres cliniques obtenus du sujet sont combinés avec une fonction de régression qui a été dérivée d'une population de prostate des sujets cancéreux.

8. Le procédé selon l'une quelconque des revendications 1 à 7, dans lequel l'échantillon biologique est obtenu à partir du sujet avant le début de la radiothérapie.

9. Le procédé selon l'une quelconque des revendications 1 à 8, dans lequel la radiothérapie est une radiothérapie radicalaire ou une radiothérapie de secours.

10. Le procédé selon l'une quelconque des revendications 1 à 9, dans lequel la prédiction de la réponse de radiothérapie est négative ou positive pour l'efficacité de la radiothérapie, dans lequel une thérapie est recommandée sur la base de la prédiction et, si la prédiction est négative, la thérapie recommandée comprend un ou plusieurs éléments parmi (i) la radiothérapie administrée plus tôt que la norme; (ii) la radiothérapie avec une dose de rayonnement accrue; (iii) une thérapie adjuvante, telle qu'une thérapie de privation androgénique; et iv) une thérapie alternative qui n'est pas une radiothérapie.

11. Un appareil pour prédire une réponse d'un cancer de la prostate soumis à une radiothérapie comprend:

- une entrée adaptée pour recevoir des données indicatives d'un profil d'expression génique pour chacun de deux ou plusieurs, par exemple 2, 3, 4 ou tous, gènes de points de contrôle immuns sélectionnés dans le groupe constitué de LAG3, LGALS9, TDO2, TMIGD2 et VTCN1, lesdits profils d'expression génique étant déterminés dans un échantillon biologique prélevé sur le sujet,
- un processeur adapté pour déterminer la prédiction de la réponse à la radiothérapie sur la base des profils d'expression génique pour les deux gènes de point de contrôle immunitaire ou plus, et
- facultativement, une unité de fourniture adaptée pour fournir la prédiction ou une recommandation de thérapie basée sur la prédiction à un soignant ou au sujet.

12. Un produit de programme informatique comprenant des instructions qui, lorsque le programme est exécuté par un ordinateur, amène l'ordinateur à exécuter une méthode comprenant:

- la réception des données indicatives d'un profil d'expression génique pour chacun de deux ou plusieurs, par exemple 2, 3, 4 ou tous, gènes de points de contrôle immuns sélectionnés dans le groupe constitué de: LAG3, LGALS9, TDO2, TMIGD2 et VTCN1, ces profils d'expression génique étant déterminés dans un échantillon biologique prélevé sur un sujet atteint d'un cancer de la prostate,
- déterminer la prédiction de la réponse à la radiothérapie sur la base des profils d'expression génique pour les deux gènes de point de contrôle immunitaire ou plus, et
- facultativement, fournir la prédiction ou une recommandation thérapeutique basée sur la prédiction à un soignant médical ou au sujet.

13. Un kit de diagnostic, comprenant:

- au moins deux amorces et/ou sondes permettant de déterminer le profil d'expression génique pour chacune de deux ou plusieurs amorces, par exemple 2, 3, 4 ou tous, gènes de points de contrôle immuns sélectionnés dans le groupe constitué de: LAG3, LGALS9, TDO2, TMIGD2 et VTCN1, dans un échantillon biologique prélevé sur le sujet, et
- un appareil tel que défini dans la revendication 11 ou un produit de programme d'ordinateur tel que défini dans la revendication 12.

14. Utilisation du kit tel que défini dans la revendication 13.

15. Utilisation du kit tel que défini dans la revendication 14 dans un procédé de prédiction d'une réponse d'un cancer de la prostate sujet à une radiothérapie.

16. Une méthode comprend:

- la réception d'un échantillon biologique prélevé sur un sujet atteint d'un cancer de la prostate,
- en utilisant le kit tel que défini dans la revendication 11 pour déterminer un profil d'expression génique pour chacun de deux ou plusieurs, par exemple 2, 3, 4 ou tous, gènes de points de contrôle immuns sélectionnés dans le groupe constitué de: LAG3, LGALS9, TDO2, TMIGD2 et VTCN1, dans l'échantillon biologique prélevé sur le sujet.

17. L'utilisation d'un profil d'expression génique pour chacun de deux ou plusieurs, par exemple 2, 3, 4 ou tous, gènes de points de contrôle immuns sélectionnés dans le groupe constitué de: LAG3, LGALS9, TDO2, TMIGD2 et VTCN1, l'invention concerne un procédé de prédiction d'une réponse d'un cancer de la prostate soumis à une radiothérapie, comprenant:

- la détermination, par un processeur, la prédiction de la réponse à la radiothérapie sur la base des profils d'expression des gènes pour les deux gènes ou plus du point de contrôle immunitaire, et
- facultativement, fournir la prédiction ou une recommandation thérapeutique basée sur la prédiction à un soignant ou au sujet.

S100 → START

S102 → OBTAIN BIOLOGICAL SAMPLES FROM FIRST SET OF PATIENTS

S104 → OBTAIN GENE EXPRESSION PROFILES FOR BIOLOGICAL SAMPLES

S106 → GENERATE REGRESSION FUNCTION

S108 → OBTAIN BIOLOGICAL SAMPLE FROM PATIENT

S110 → OBTAIN GENE EXPRESSION PROFILE FOR BIOLOGICAL SAMPLE

S112 → COMPUTE PREDICTION FOR PATIENT WITH REGRESSION FUNCTION

S114 → PROVIDE THERAPY RECOMMENDATION FOR THE PATIENT BASED ON THE PREDICTION

S116 → END

FIG. 1

FIG. 2

FIG. 3

FIG. 4

FIG. 5

FIG. 6

FIG. 7

FIG. 8

EP 4 127 243 B1

FIG. 9

FIG. 10

EP 4 127 243 B1

**FIG. 11**

EP 4 127 243 B1

FIG. 12

FIG. 13

FIG. 14

FIG. 15

FIG. 16

FIG. 17

FIG. 18

EP 4 127 243 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019028285 A2

### Non-patent literature cited in the description

- **BRAY F. et al.** Global cancer statistics 2018: GLOBOCAN estimates of incidence and mortality worldwide for 36 cancers in 185 countries. *CA Cancer J Clin,* 2018, vol. 68 (6), 394-424
- *Cancer Facts & Figures 2010,* 2010
- **GRIMM P. et al.** Comparative analysis of prostate-specific antigen free survival outcomes for patients with low, intermediate and high risk prostate cancer treatment by radical therapy. Results from the Prostate Cancer Results Study Group. *BJU Int,* 2012, (1), 22-29
- **DAL PRA A. et al.** Contemporary role of postoperative radiotherapy for prostate cancer. *Transl Androl Urol,* 2018, vol. 7 (3), 399-413
- **HERRERA F.G. ; BERTHOLD D.R.** Radiation therapy after radical prostatectomy: Implications for clinicians. *Front Oncol,* 2016, vol. 6 (117
- **PISANSKY T.M. et al.** Salvage radiation therapy dose response for biochemical failure of prostate cancer after prostatectomy - A multi-institutional observational study. *Int J Radiat Oncol Biol Phys,* 2016, vol. 96 (5), 1046-1053
- **RESNICK M.J. et al.** Long-term functional outcomes after treatment for localized prostate cancer. *N Engl J Med,* 2013, vol. 368 (5), 436-445
- **HEGARTY S.E. et al.** Radiation therapy after radical prostatectomy for prostate cancer: Evaluation of complications and influence of radiation timing on outcomes in a large, population-based cohort. *PLoS One,* 2015, vol. 10 (2
- **PARAVATI A.J. et al.** Variation in the cost of radiation therapy among medicare patients with cancer. *J Oncol Pract,* 2015, vol. 11 (5), 403-409
- **HALL W.A. et al.** Biomarkers of outcome in patients with localized prostate cancer treated with radiotherapy. *Semin Radiat Oncol,* 2016, vol. 27, 11-20
- **RAYMOND E. et al.** An appraisal of analytical tools used in predicting clinical outcomes following radiation therapy treatment of men with prostate cancer: A systematic review. *Radiat Oncol,* 2017, vol. 12 (1), 56

- **DEN R.B. et al.** Genomic Classifier Identifies Men With Adverse Pathology After Radical Prostatectomy Who Benefit From Adjuvant Radiation Therapy. *Journal of Clinical Oncology,* 2015, vol. 33 (8), 944-951
- **GRASSBERGER C. et al.** Assessing the interactions between radiotherapy and antitumour immunity. *Nature Reviews Clinical Oncology,* 2019, vol. 16 (12), 729-745
- **MANTOVANI A. et al.** Cancer-related inflammation. *Nature,* 2008, vol. 454 (7203), 436-444
- **GIRALDO N.A. et al.** The clinical role of the TME in solid cancer. *Br J Cancer,* 2019, vol. 120 (1), 45-53
- **SHIAO S.L. et al.** Regulation of prostate cancer progression by tumor microenvironment. *Cancer Lett,* 2016, vol. 380 (1), 340-348
- **BARKER H.E. et al.** The tumor microenvironment after radiotherapy: Mechanisms of resistance or recurrence. *Nat Rev Cancer,* 2015, vol. 15 (7), 409-425
- **LI H. et al.** The immune checkpoint regulator PDL1 is an independent prognostic biomarker for biochemical recurrence in prostate cancer patients following adjuvant hormonal therapy. *J Cancer,* 2019, vol. 10 (14), 3102-3111
- **TAGHIZADEH H. et al.** Immune checkpoint inhibitors in mCRPC - Rationales, challenges and perspectives. *Oncoimmunology,* 2019, vol. 8 (11
- **GROSSO J.F. et al.** LAG-3 regulates CD8+ T cell accumulation and effector function in murine self- and tumor-tolerance systems. *J Clin Invest,* 2007, vol. 117 (11), 3383-3392
- **LONG L. et al.** The promising immune checkpoint LAG-3: From tumor microenvironment to cancer immunotherapy. *Genes Cancer,* 2018, vol. 9 (5-6), 176-189
- **NORSTRÖM M.M. et al.** Progression of benign prostatic hyperplasia is associated with pro-inflammatory mediators and chronic activation of prostate-infiltrating lymphocytes. *Oncotarget,* 2016, vol. 7 (17), 23581-23593

- **HEUSSCHEN R. et al.** Endothelial LGALS9 splice variant expression in endothelial cell biology and angiogenesis. *Biochim Biophys Acta,* 2014, vol. 1842 (2), 284-292
- **LADERACH D.J. et al.** A unique galectin signature in human prostate cancer progression suggests galectin-1 as a key target for treatment of advanced disease. *Cancer Res,* 2013, vol. 73 (1), 86-96
- **ITOH A. et al.** Galectin-9 induces atypical ubiquitination leading to cell death in PC-3 prostate cancer cells. *Glycobiology,* 2019, vol. 29 (1), 22-35
- **NOBUMOTO A. et al.** Galectin-9 suppresses tumor metastasis by blocking adhesion to endothelium and extracellular matrices. *Glycobiology,* 2008, vol. 18 (9), 735-744
- **CHEONG J.E. ; SUN L.** Targeting the IDO1/TDO2-KYN-AhR pathway for cancer immunotherapy - Challenges and opportunities. *Trends Pharmacol Sci,* 2018, vol. 39 (3), 307-325
- **PILOTTE L. et al.** Reversal of tumoral immune resistance by inhibition of tryptophan 2,3-dioxygenase. *Proc Natl Acad Sci USA,* 2012, vol. 109 (7), 2497-2502
- **DING Y. et al.** Gene expression differences in prostate cancers between young and old men. *PLoS Genet,* 2016, vol. 12 (12
- **RAHIMI N. et al.** Identification of IGPR-1 as a novel adhesion molecule involved in angiogenesis. *Molec Biol Cell,* 2012, vol. 23 (9), 1646-1656
- **JANAKIRAM M. et al.** HHLA2 and TMIGD2: New immunotherapeutic targets of the B7 and CD28 families. *Oncoimmunology,* 2015, vol. 4 (8
- **PODOJIL J.R. ; MILLER S.D.** Potential targeting of B7-H4 for the treatment of cancer. *Immunol Rev,* 2017, vol. 276 (1), 40-51
- **MACGREGOR H.L. ; OHASHI P.S.** Molecular pathways: Evaluating the potential for B7-H4 as an immunoregulatory target. *Clin Cancer Res,* 2017, vol. 23 (12), 2934-2941
- **LI J. et al.** Co-inhibitory molecule B7 superfamily member 1 expressed by tumor-infiltrating myeloid cells induces dysfunction of anti-tumor CD8+ T cells. *Immunity,* 2018, vol. 48 (4), 773-786
- **COOPERBERG M.R. et al.** The CAPRA-S score: A straightforward tool for improved prediction of outcomes after radical prostatectomy. *Cancer,* 2011, vol. 117 (22), 5039-5046